# EUROPEAN PATENT SPECIFICATION

(11) **EP 1 592 388 B1**
(45) Date of publication and mention of the grant of the patent: **21.04.2010**
(21) Application number: 04705647.8
(22) Date of filing: 27.01.2004
(51) Int. Cl.: G01N 33/53, G01N 33/567

(54) **METHODS FOR DIAGNOSIS AND MONITORING OF NEUROLOGICAL DISEASE BY DETECTION OF AN ENCEPHALOTOXIN**
VERFAHREN ZUR DIAGNOSE UND ÜBERWACHUNG VON NEUROLOGISCHEN ERKRANKUNGEN DURCH NACHWEIS EINES ENCEPHALOTOXINS
PROCEDES POUR LE DIAGNOSTIC ET LA SURVEILLANCE D'UNE MALADIE NEUROLOGIQUE PAR DETECTION D'UNE ENCEPHALOTOXINE

(30) Priority: 27.01.2003 US 443219 P
(43) Date of publication of application: 09.11.2005
(73) Proprietor: Jacobus Pharmaceutical Company, Inc., Princeton, NJ 08540 (US)
(72) Inventor: GIULIAN, Dana, J., Houston, TX 77054 (US)
(74) Representative: W.P. Thompson & Co.
(86) International application number: PCT/US2004/002236
(87) International publication number: WO 2004/066943

(56) References cited:
- US-A- 6 043 283
- ANDERSON ERIC ET AL: "HIV-1-associated dementia: a metabolic encephalopathy perpetrated by virus-infected and immune-competent mononuclear phagocytes." JOURNAL OF ACQUIRED IMMUNE DEFICIENCY SYNDROMES (1999) 1 OCT 2002, vol. 31 Suppl 2, 1 October 2002 (2002-10-01), pages S43-S54, XP002485132 ISSN: 1525-4135
- JIANG Z G ET AL: "Glutamate is a mediator of neurotoxicity in secretions of activated HIV-1-infected macrophages." JOURNAL OF NEUROIMMUNOLOGY 2 JUL 2001, vol. 117, no. 1-2, 2 July 2001 (2001-07-02), pages 97-107, XP002485133 ISSN: 0165-5728
- GIULIAN D ET AL: "The envelope glycoprotein of human immunodeficiency virus type 1 stimulates release of neurotoxins from monocytes." PROCEEDINGS OF THE NATIONAL ACADEMY OF SCIENCES OF THE UNITED STATES OF AMERICA 1 APR 1993, vol. 90, no. 7, 1 April 1993 (1993-04-01), pages 2769-2773, XP002485134 ISSN: 0027-8424
- GIULIAN DANA ET AL: "Phagocytic microglia release cytokines and cytotoxins that regulate the survival of astrocytes and neurons in culture" NEUROCHEMISTRY INTERNATIONAL, vol. 25, no. 3, 1994, pages 227-233, XP002485135 ISSN: 0197-0186
- GIULIAN DANA ET AL: "Inflammatory glia mediate delayed neuronal damage after ischemia in the central nervous system" STROKE, AMERICAN HEART ASSOCIATION, DALLAS TX, US, vol. 24, no. 12 Suppl, 1 January 1993 (1993-01-01), pages I84-I90, XP008093326 ISSN: 0039-2499
- GIULIAN D.: 'Neurogenetics '99: Microglia and the Immune Pathology of Alzheimer Disease' AM. J. HUM. GENET. vol. 65, November 1999, pages 13 - 18, XP008096682
- GIULIAN D. ET AL.: 'Senile Plaques Stimulate Microglia to Release: a Neurotoxin found in Alzheimer Brain' NEUROCHEM. INT. vol. 27, no. 1, 1995, pages 119 - 137, XP008096698
- GIULIAN D. ET AL.: 'Specific domains of b-amyloid from Alzheimer plaques elicit neuron killing in human microglia' J. NEUROSCI. vol. 16, 1996, pages 6021 - 6037, XP008096727
- KITAGAWA E; TAKAHASHI J; MOMOSE Y; IWAHASHI H: "Effects of the pesticide thiuram: Genome-wide screening of indicator genes by yeast DNA microarray" ENVIRONMENTAL SCIENCE AND TECHNOLOGY, vol. 36, no. 18, 15 September 2002 (2002-09-15), pages 3908-3915, USA

## Description

### REFERENCE TO GOVERNMENT GRANTS

Portions of the disclosure herein may have been supported in part by grants from the National Institutes of Health, Grant No. AG12548. The United States Government may have certain rights in this application.

### FIELD OF THE INVENTION

The invention relates to the correlation of clinical manifestations of neurological disease with a neurotoxin produced by affected brain mononuclear phagocytes. The invention also relates to methods for diagnosing a neurological disease or risk for loss of cognition by detecting a neurotoxin in a biological sample of a subject. The neurotoxin, encephalotoxin, has been found to be released by an inflammatory cascade that chronically damages neurons in neurological disease, for example, HIV-1-associated dementia (HAD), neuro-AIDS, Creutzfeld-Jakob Disease, Mild Cognitive Impairment, prion disease, mild cognitive/ motor dysfunction, acute stroke, acute trauma, and Alzheimer's disease (AD). The inflammatory cascade involves activation of mononuclear phagocytes and loss of synaptic connections and neurons, thus resulting in a decline in information processing, attention, learning, and information retrieval with overall loss of intellectual function.

### BACKGROUND OF THE INVENTION

Loss of cognition and dementia associated with neurological disease results from damage to neurons and synapses that serve as the anatomical substrata for memory, learning, and information processing. Despite much interest, biochemical pathways responsible for progressive neuronal loss in these disorders have not been elucidated.

Alzheimer's disease (AD) accounts for more than 15 million cases worldwide and is the most frequent cause of dementia in the elderly (Terry, R.D. *et al.* (eds.), ALZHEIMER'S DISEASE, Raven Press, New York, 1994). AD is thought to involve mechanisms which destroy neurons and synaptic connections. The neuropathology of this disorder includes formation of senile plaques which contain aggregates of Aβ1-42 (Selkoe, Neuron, 1991, 6:487-498; Yankner et al., New Eng. J. Med., 1991, 325:1849-1857; Price et al., Neurobiol. Aging, 1992, 13, 623-625; Younkin, Ann. Neurol., 1995, 37:287-288). Senile plaques found within the gray matter of AD patients are in contact with reactive microglia and are associated with neuron damage (Terry et al., "Structural Basis of the Cognitive Alterations in Alzheimer Disease", ALZHEIMER's DISEASE, NY, Raven Press, 1994, Ch. 11, 179-196; Terry, R.D. et al. (eds.), ALZHEIMER'S DISEASE, Raven Press, New York, 1994; Perlmutter et al., J. Neurosci. Res., 1992, 33:549-558). Plaque components from microglial interactions with Aβ plaques tested *in vitro* were found to stimulate microglia to release a potent neurotoxin, thus linking reactive microgliosis with AD neuronal pathology (Giulian et al., Neurochem. Int., 1995, 27:119-137).

Several lines of evidence now support the concept that microglia-derived neurotoxins contribute to AD pathology. First, microglia-derived toxins can be extracted from AD brain regions laden with plaques but not from identical brain regions in age-matched control or ALS brain tissues (Giulian et al. (1995) Neurochem. Int., 27: 119-137; Giulian et al. (1996) J. Neurosci., 16: 6021-6037). Second, regional distributions of toxic activity show the greatest concentrations of microglia-derived neuron poisons in neocortical tissues and hippocampi of AD (vs. controls or ALS), areas containing large numbers of reactive microglia. In contrast, cerebellum, white matter, and neocortical tissues from normal or ALS patients, which had few, if any, reactive microglial clusters, show little neurotoxic activity. Moreover, the relative number of reactive microglial clusters in each brain region is significantly correlated to the level of neurotoxic activity extracted from that region (p<0.005). Third, isolated plaque fragments or synthetic human Aβ11-40 or Aβ1-42 peptides are found to activate human microglia to release neurotoxins in culture (Giulian et al. (1995) Neurochem. Int., 27: 119-137; Giulian et al. (1996) J. Neurosci., 16: 6021-6037). No neurotoxic effects, however, are detected when plaques or peptides were placed directly atop neurons or when microglia are exposed to fractions lacking plaques isolated from AD, ALS, or normal, aged control brains (Giulian et al. (1995) Neurochem. Int. 27: 119-137; Giulian et al. (1996) J. Neurosci. 16: 6021-6037). Thus, the toxic effects of isolated plaques on neurons are indirect and mediated by a neurotoxic activity released from plaque-stimulated microglia. Fourth, there is neurotoxic activity found in CSF from AD patients, but not detected in samples from disease controls (U.S. Patent 6,043,283 to Giulian; Giulian et al. (1999) Am. J. Hum. Genet., 65:13-18). Fifth, infusion of Aβ-coupled microspheres into hippocampus produces inflammatory responses at the site of infusion in rats (U.S. Patent No. 6,043,283 to Giulian). Together, these data indicate that plaque-activation of microglia through contact with Aβ peptides produces neuron-killing factors in discrete areas of AD brain (Giulian et al. (1995) Neurochem. Int., 27: 119-137).

Although most patients developing AD will go through a transient period of mild cognitive impairment (MCI), they will often not present to a physician during this early phase of the disease. There is a consensus among research groups that subjects with MCI are at increased risk for progressing to AD (Grundman et al. (1996) Neurology 46:403; Flicker et al. (1991) Neurology 41:1006-1009; Masur et al. (1994) Neurology 44:1427-1432; Tierney et al. (1996) Neurology 46:149-154). Memory impairment is commonly the most prominent feature of MCI but might include other patterns including defects primarily in language or visuomotor performance (Hughes et al. (1982) Br. J. Psychiatry, 140:566-572; Berg (1988) Psychopharmacol. Bull., 24:637-639; Morris (1993) Neurology, 43:2412-2414; Rubin et al. (1989) Arch. Neurol., 46:379-382; Grundman et al. (1996) Neurology, 46:403; Flicker et al. (1991) Neurology, 41:1006-1009; Masur et al. (1994) Neurology, 44:1427-1432;Tierney et al. (1996) Neurology, 46:149-154). Attempts at characterizing mild cognitive impairment have been carried out using the Clinical Dementia Rating (CDR) Scale, which rates the severity of dementia as absent, mild, moderate, or severe. Rubin et al. ((1989) Arch. Neurol., 46:379-382) concluded that individuals with a CDR of 0.5 likely have "very mild" AD in the majority of cases [The CDR 0.5 classification is characterized by consistent forgetfulness, which is mild with little if any impairment in other functions such as orientation, community affairs, home, and hobbies, judgment, and personal care.] Other measures also have been used to identify MCI subjects. For example, poor delayed recall has been shown to be the best predictor of progression, the best predictor of subsequent dementia in non demented elderly subjects, and the best discriminator between normal aging and mild AD (Flicker et al. (1991) Neurology, 41:1006-1009; Masur et al. (1994) Neurology, 44:1427-1432;Tierney et al. (1996) Neurology, 46:149-154). The time required for subjects with MCI to develop a clinical diagnosis of AD has been estimated by the Alzheimer's Disease Cooperative Study (ADCS) at about 30% at 2 years and 45% at 3 years.

HIV-1 infection and neuro-AIDS produce devastating effects upon the brain and spinal cord. Although the underlying anatomical basis for impaired cognition during HIV-1 infection remains obscure, there is a reduction of up to 40% of large neurons scattered throughout the neocortex in advanced disease with dementia (Masliah et al. (1992) J. Neuropath Exp Neurol., 51: 585-593) and a striking early loss of synapses (Asare et al. (1996) Am J Path 148: 31-38; Everall et al. (1993) J. Neuropath. Exp. Neurol. 52: 561-566).

HIV-1 associated dementia (HAD) is characterized by cognitive dysfunction, declining motor performance, and behavioral changes. It occurs primarily in the more advanced stages of HIV infection when CD4 cell counts are relatively low. While the progression of dysfunction is variable, it is regarded as a serious complication with fatal outcome. The diagnosis of cognitive loss due to HIV is by process of exclusion -- no approved marker exists to monitor HIV-specific injury to the CNS. Without such a marker, there are no clinical indications to evaluate patients until significant functional loss appears and there are few opportunities to develop new treatment strategies to prevent HIV brain damage. Therefore, it is very desirable to identify patients at early pre-symptomatic stages.

Prior to HAART (defined here as combination therapy using 3 or more anti-retroviral agents), 60% of those with AIDS developed dementia. This incidence appears to have fallen to about 10 to 15%, but cognitive dysfunction remains a problem for over half of the HIV/AIDS population (Giulian et al. (1990) Science, 250: 1593-1596; Giulian et al. (1993) Proc. Natl. Acad. Sci., 90:2769-2773; Giulian (1995) In: NEUROGLIA (H Kettenmann, B Ransom Eds) Oxford University Press, pp. 671-684; Giulian et al. (1998) In: INFLAMMATORY MECHANISMS OF NEURODEGENERATION AND ITS MANAGEMENT (P. Wood, ed.); Humana Press, Vol 4, pp. 109-128).

HIV-1 brain pathology involves diffuse synaptic damage in the neocortex, the loss of cortical neurons, and a population of infected, reactive mononuclear phagocytes, including invading blood monocytes, microglia, and multi-nucleated giant cells. These giant cells represent a fusion of HIV-infected mononuclear phagocytes that are coated with gp120, the retroviral envelope protein; presence of giant cells has been correlated with cognitive impairment during HIV-1 infection. Currently, most research groups in the field agree that poisons released by infected mononuclear phagocytes are a primary cause of cognitive loss in the HIV-1(+) population (Vitokovic et al. (1998) Medical Sciences, 321: 1015-1021; Morgello et al. (2001) Neuropath. App. Neurobiol., 27: 326-335; Lawrence et al.. (2002) Microbes and Infection, 4: 301-308; Masliah et al. (1992) J. Neuropath. Exp. Neurol., 51: 585-593; Maslliah et al. (1995) J. Neuropath. and Exp. Neurol., 54: 350-357; Asare et al. (1996) Ant. J. Path., 148: 31-38; Everall et al. (1993) J. Neuropath. Exp. Neurol., 52: 561-566).

Several lines of evidence now support the concept that mononuclear phagocyte-derived neurotoxins contribute to the neuron injury within brain during HIV-1 infection. First, HIV-1 neither infected neurons nor showed a direct toxic effect upon neurons (Giulian et al. (1996) J. Neurosci., 16:3139-3153, Giulian et al. (1990) Science 250: 1593-1596; Levine et al. (1976) Biochim. Biophys. Acta, 452: 458-467). Second, HIV-1 mononuclear phagocytes (THP-1, U937, human blood monocytes, and human brain microglia) released neurotoxins when infected *in vitro* with HIV-1; in contrast, lymphocytes (H9, human blood lymphocytes) did not (Giulian et al. (1996) J. Neurosci., 16:3139-3153; Giulian et al. (1990) Science, 250: 1593-1596). Third, human mononuclear phagocytes (blood monocytes and microglia) isolated from infected donors released the same neurotoxin as recovered from *in vitro* experiments; again, isolated infected lymphocytes did not (Giulian et al. (1996) J. Neurosci., 16:3139-3153). Fourth, neurotoxic activity can be recovered from brain tissues of infected individuals (Giulian et al. (1993) Proc. Natl. Acad. Sci., 90:2769-2773; Giulian (1995) In: NEUROGLIA (H Kettenmann, B Ransom, Eds,) Oxford University Press, pp. 671-684; Giulian et al. (1998) In: INFLAMMATORY MECHANISMS OF NEURODEGENERATION AND ITS MANAGEMENT (P. Wood, ed.); Humana Press, Vol 4, pp. 109-128). Fifth, gp120, the viral envelope glycoprotein, can stimulate neurotoxin release from human blood monocytes and microglia; other viral proteins including tat did not (Levine et al. (1976) Biochim. Biophys. Acta, 452: 458-467). Sixth, high concentrations of neurotoxin were found in the cerebrospinal fluid of HIV-1(+) individuals. And seventh, a family of neurotoxic heparan oligosaccharides can be isolated from HIV-1 infected cells and from HIV CSF.

Although reactive mononuclear phagocytes release a number of bio-active substances, few of these compounds are actually able to harm neurons at concentrations found to exist in neurodegenerative disease (Hardy et al. (2002) Science, 297:353; Mourdian et al., (1989) Neurosci. Lett., 105: 233; Milstein et al. (1994) J. Neurochemistry, 63, 1178; Giulian et al. (1990) Science, 250:1593). Moreover, few of such candidate neuron poisons are present in both AD and HAD. For example, increased tissue concentrations of "toxic" forms of Aβ1-42 are characteristic for AD (Hardy et al. (2002) Science, 297:353), but do not occur in HAD. Similarly, elevated quinolinic acid levels occur in the cerebrospinal fluid (CSF) of subjects with HAD (Mourdian et al. (1989) Neurosci. Lett., 105:233), but not in those with AD (Milstein, et al. (1994) J. Neurochemistry, 63: 1178). In contrast, both AD and HAD brain tissues contain a heterogeneous group of shall stable molecules with potent neurotoxic actions (Giulian et al. (1990) Science, 250:1593; Giulian et al. (1995) Neurochem. Int., 27:119; Giulian et al. (1996) J. Neuroscience 16: 6021). Cultured mononuclear phagocytes activated by exposure to amyloid plaques, synthetic β-amyloid peptides, HIV-1, or gp120, produce these same neurotoxins (Giulian, et al. (1993) Proc. Natl. Acad. Sci. USA, 90: 2769; Giulian et al. (1998) J. Biol. Chem., 273: 29719). Such observations suggest that a common, though unidentified, pathway mediates immune-driven neuron pathology in both AD and HAD.

US6,043,283 discloses a method for using tyramine compounds to inhibit the toxic effects of neurotoxins, to treat and diagnose neuron degenerative diseases and disorders and to identify compounds that inhibit the toxic effects of neurotoxins.

Anderson, Eric et al; Journal of Acquired Immune Deficiency Syndromes; Vol 31, Supp 2, 2002, S43 to S 54, discloses that H1V-1-associated dementia is a metabolic encephalopathy caused by productive viral infection of brain mononuclear phagocytes and sustained by paracrine-amplified, inflammatory, neurotoxic responses.

Jiang, Z-G et al; Journal of Neuroinnunology, Vol 117, 2001, 97 to 107, gives details of neurotoxins secreted by HIV-1-infected mononuclear phagocytes contributing to the pathophysiology of HIV-1-associated dementia.

Giulian, Dana, et al; Proc. Natl. Acad. Sci. USA; 90,1993, 2769 to 2773, discloses the production of soluble factors that kill neurons in culture by mononuclear phagocytes infected with human immunodeficiency virus 1.

Giulian, Dana, et al; Nurochemistry International, Vol. 25, Number 3, 1994, 227 to 233, discloses reactive microgila mediating both astrogliosis and neuronal injury through the simultaneous release of cell growth factors and poisons, and that the net effect of secretion products from phagocytic microglia is to diminish neuronal survival.

Giulian, Dana, et al; Stroke, American Heart Association, Vol 24, Number 12, Supp 1, 1993, 184 to 190, discloses that reactive microglia respond within hours to central nervous system ischemic injury, but that it is at least several days after an insult, before these activated mononuclear phagocytes reach a peak of secretary activity with the release of neurotoxins, and that this period of cytotoxic secretion is associated with a delayed neuronal loss seen in tissues in neighbouring sites of ischemia.

As the clinical expression of neurological disease may occur only after a significant degree of neuron loss and synaptic damage beyond a critical threshold necessary for adequate adaptive function, early pre-symptomatic detection of disease pathology offers the opportunity to slow disease progression. The present invention provides methods for diagnosis of neurological disease and risk for loss of cognition, including, for example, Alzheimer's disease, HIV-1 associated dementia (HAD), neuro-AIDS, Creutzfeld-Jakob disease, Mild Cognitive Impairment (MCI), prion disease, mild cognitive/ motor dysfunction, acute stroke, or acute trauma. The methods of the invention allow early detection of neurological disease and risk for loss of cognition, thereby allowing earlier intervention in the progression of disease. Also provided are methods for monitoring the progression and treatment of neurological disease by monitoring encephalotoxin levels in a subject.

### SUMMARY OF THE INVENTION

The present invention provides a method of diagnosing a neurological disease or risk for loss of cognition in a subject comprising detecting an encephalotoxin in a biological sample comprising cerebrospinal fluid, spinal cord tissue or brain tissue of said subject, wherein said detecting comprises contacting said biological sample of said subject with neurons both in the presence of and in the absence of an encephalotoxin inactivator and comparing neuron survival in the presence of said encephalotoxin inactivator relative to neuron survival in the absence of said encephalotoxin inactivator, a decrease in neuron survival in the absence of said encephalotoxin inactivator being indicative of said neurological disease, the encephalotoxin being an oligosaccharide comprising at least one glucosamine having N-sulfation and O6-sulfation; said encephalotoxin lacking peptide bonds; and said encephalotoxin having a molecular weight of less than 2000 daltons; the encephalotoxin inactivator being heparin lyase I, N-sulfaminidase, glucosamine-6-sulfatase, or nitrous acid solution.

The present invention further provides a method of diagnosing a neurological disease or risk for loss of cognition in a subject comprising detecting an encephalotoxin in a biological sample comprising cerebrospinal fluid, spinal cord tissue or brain tissue of said subject, wherein said step of detecting comprises comparing light absorbance of said biological sample in the presence of an encephalotoxin inactivator to light absorbance of said biological sample in the absence of said encephalotoxin inactivator, said light absorbance being measured following HPLC adsorption chromatography, and an increased absorbance in the absence of said encephalotoxin inactivator being indicative of said neurological disease, the encephalotoxin being an oligosaccharide comprising at least one glucosamine having N-sulfation and O6-sulfation, lacking peptide bonds, and having a molecular weight of less than 2000 daltons.

Neurological diseases that may be diagnosed or monitored by the methods of the invention include neurodegenerative and neuro-inflammatory diseases and disorders such as, but not limited to, Alzheimer's Disease, Creutzfeldt-Jakob Disease, Human Immunodeficiency Vims-1 (HIV-1)-associated dementia (HAD), Mild Cognitive Impairment (MCI), prion disease, mild cognitive/ motor dysfunction, acute stroke, acute trauma, and neuro-AIDS. In various embodiments, the methods of the invention may be used in the diagnosis or monitoring of human, primate, bovine, equine, canine, feline, porcine, or rodent subjects.

In some embodiments of the invention, comparison of neuron survival comprises comparison of the ED₅₀ of the encephalotoxin in the presence of the encephalotoxin inactivator relative to the ED₅₀ of the encephalotoxin in the absence of the encephalotoxin inactivator, wherein a lower ED₅₀ of the encephalotoxin in the absence of encephalotoxin inactivator relative to the ED₅₀ of the encephalotoxin in the presence of encephalotoxin inactivator is indicative of neurological disease or risk for loss of cognition.

The present invention further provides a method of monitoring treatment of a neurological disease in a subject comprising comparing encephalotoxin level in a first and second biological sample of said subject, said first and second biological samples each comprising cerebrospinal fluid, spinal cord tissue or brain tissue, wherein said first biological sample is taken from said subject at an earlier timepoint than said second biological sample, wherein said second biological sample is taken from said subject following treatment of said neurological disorder, and wherein said encephalotoxin level is measured by light absorbance of said biological sample, said light absorbance being measured following HPLC adsorption chromatography, and an increased light absorbance of said second biological sample being indicative of progression of said neurological disease, the encephalotoxin being an oligosaccharide comprising at least one glucosamine having N-sulfation and O6-sulfation; said encephalotoxin lacking peptide bonds; and said encephalotoxin having a molecular weight of less than 2000 daltons.

In some embodiments, the first biological sample is taken from such subject following treatment of said neurological disease.

A further embodiment of the invention provides a method of monitoring progression of neurological disease in a subject comprising detecting an increase in encephalotoxin level in said subject over time, wherein said step of detecting comprises measuring an increased light absorbance of an encephalotoxin in a first biological sample of said subject relative to light absorbance of an encephalotoxin of a second biological sample of said subject, wherein said first and second biological samples each comprise cerebrospinal fluid, spinal cord tissue or brain tissue, and second biological sample is taken from said subject before said first biological sample, wherein said light absorbance is measured following HPLC adsorption chromatography, wherein said encephalotoxin comprises an oligosaccharide comprising at least one glucosamine having N-sulfation and 06-sulfation, lacks peptide bonds, and has a molecular weight of less than 2000 daltons, said increased light absorbance being indicative of progression of said neurological disease.

Preferably, light absorbance is measured at a wavelength of 232 nanometers

Also provided by the invention is a method for monitoring progression of neurological disease in a subject comprising detecting an increase in encephalotoxin level in said subject over time, wherein said step of detecting comprises contacting a first biological sample of said subject with neurons, contacting a second biological sample of said subject with neurons, wherein said first and second biological samples each comprise cerebrospinal fluid, spinal cord tissue or brain tissue; and detecting decreased neuron survival in the presence of said second biological sample, wherein said second biological sample is taken at a later timepoint than said first biological sample; wherein said encephalotoxin comprises an oligosaccharide comprising at least one glucosamine having N-sulfation and O6-sulfation, lacks peptide bonds, and has a molecular weight of less than 2000 daltons; and wherein said decreased neuron survival is indicative of progression of said neurological disease.

In some embodiments of the invention, one of the biological samples is taken during the prodromic phase of said neurological disease.

The invention further provides a method of monitoring treatment of a neurological disease in a subject comprising detecting an increase in encephalotoxin level in said subject over time, wherein said step of detecting comprises contacting a first biological sample of said subject with neurons, contacting a second biological sample of said subject with neurons, wherein said first and second biological samples each comprise cerebrospinal fluid, spinal cord tissue or brain tissue, and detecting decreased neuron survival in the presence of said second biological sample, wherein said second biological sample is taken at a later timepoint than said first biological sample and following treatment of said neurological disease; wherein said encephalotoxin comprises an oligosaccharide comprising at least one glucosamine having N-sulfation and 06-sulfation, lacks peptide bonds, and has a molecular weight of less than 2000 daltons; and wherein said decreased neuron survival is indicative of progression of said neurological disease.

### BRIEF DESCRIPTION OF THE DRAWINGS

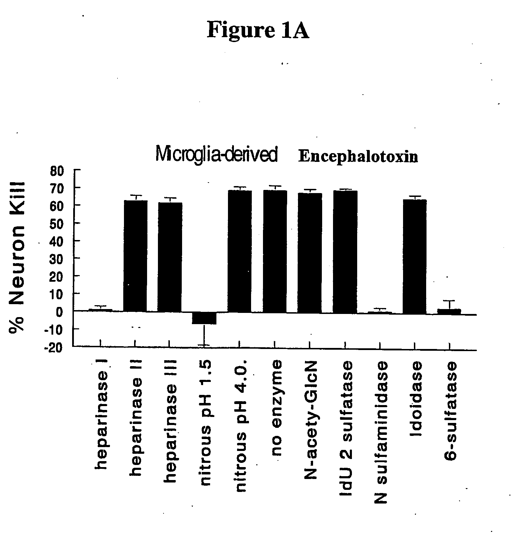

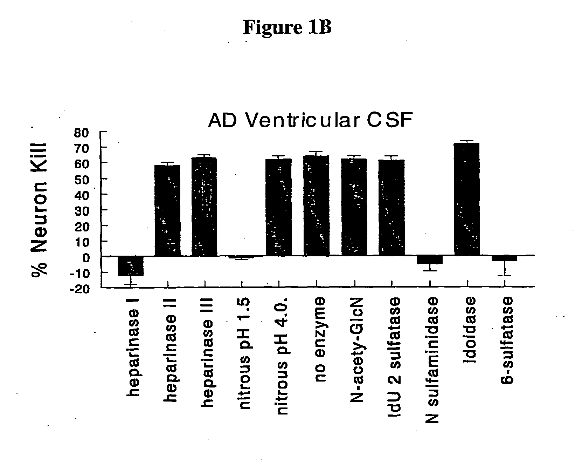

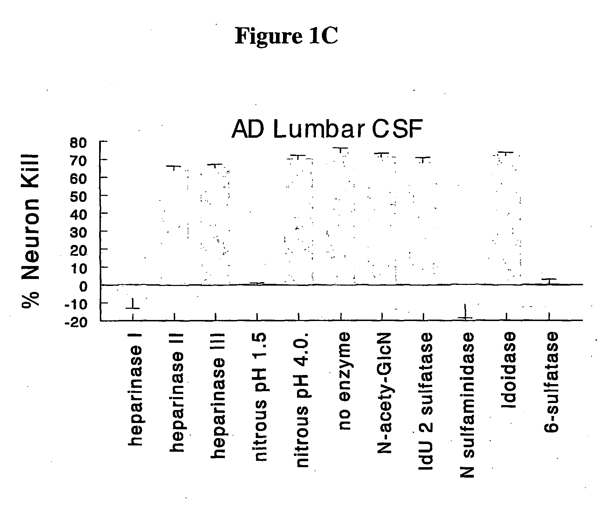

Figure 1 illustrates inactivation of encephalotoxin by various methods specific for heparan sulfate and heparin. As shown in Fig. 1A, encephalotoxin released by BV2 microglia was inactivated by nitrous acid pH 1.5, by heparin lyase I (E.C. 4.2.2.7), and by sulfatases that cleave at O-6 and from N-sulfated glucosamine (GlcNS) (glucosamine-6-sulfatase (E.C. 3.1.6.14) and N-sulfaminidase (E.C. 3.10.1.1)). As shown in Fig. 1B, encephalotoxin found in ventricular CSF of AD brain was inactivated by nitrous acid pH 1.5, by heparin lyase I, and by sulfatases that cleave at O-6 and from GlcNS. As demonstrated in Figure 1C, encephalotoxin recovered from lumbar CSF of subject with AD was inactivated by nitrous acid pH 1.5, by heparin lyase I, and by sulfatases that cleave at O-6 and from GlcNS.

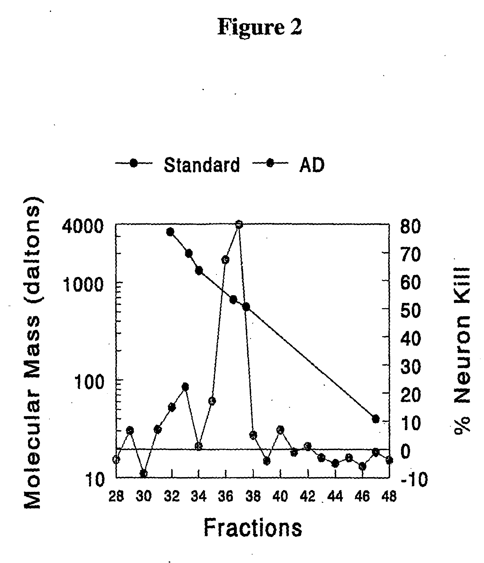

**Figure 2** illustrates the determination of molecular mass of encephalotoxin using a TSK-GW2500PXL with a linear sieving range from 300 to 3000 daltons. Commercially available heparan oligomers were used as standards. CSF samples (100 µl) from probable AD showed a minor peak and major peak of neurotoxic activity that range in size from about 700 to 1,900 daltons. These estimated molecular masses suggest that at least some forms of encephalotoxin comprise about 4 to 8 saccharide residues.

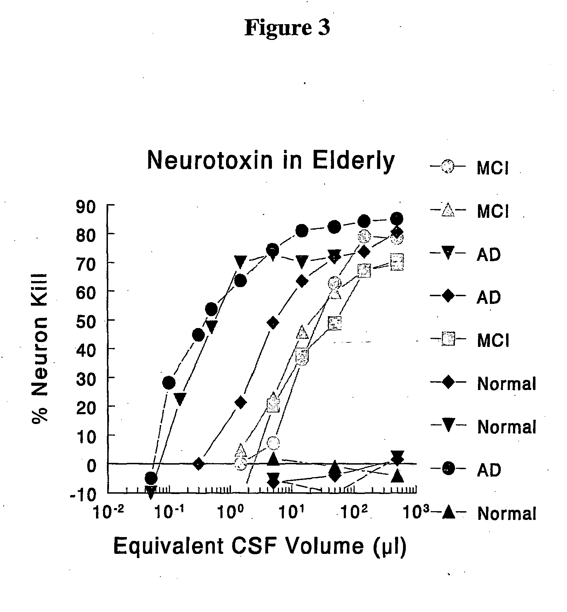

**Figure 3** shows dose response curves for encephalotoxin isolated from probable AD, MCI, and normal elderly subjects. Increasing amounts of toxin are found in those subjects with greater cognitive impairment.

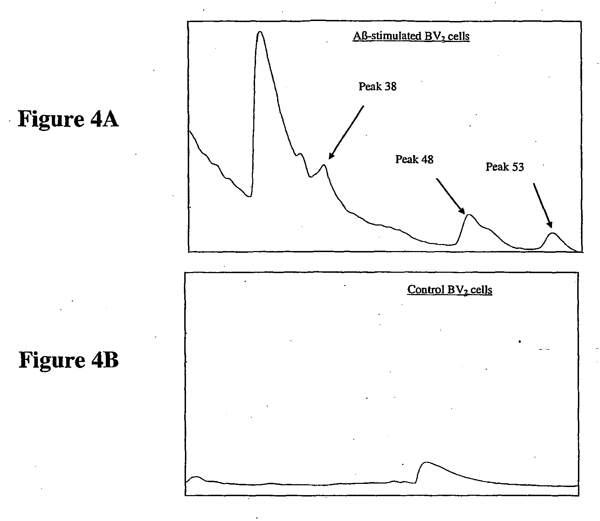

**Figure 4A** shows the results of anion-exchange HPLC (ProPAK PA1, 0.0 to 0.7 M NaCl, UV @ 232 nm) separation of encephalotoxin from microglial BV2 cells stimulated with Aβ1-42. Three peaks (PEAK 38, 48, and 53) corresponding to the encephalotoxin were detected. The encephalotoxin of PEAKS 38, 48, and 53 was 1) sensitive to heparin lyase 1, 2) sensitive to nitrous acid pH 1.5 and 3) toxic to hippocampal neurons (data not shown). As demonstrated in **Figure 4B****,** these same peaks were absent from conditioned media recovered from control BV2 cells that were not exposed to Aβ1-42.

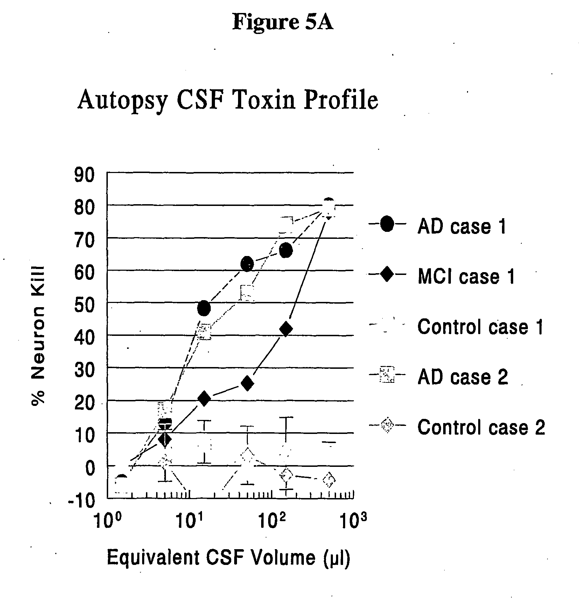

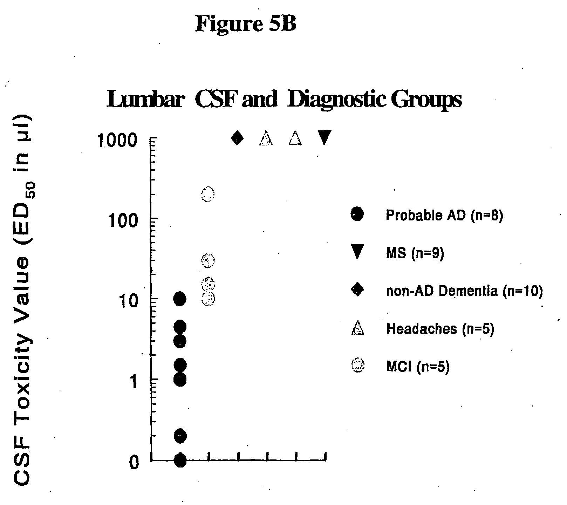

**Figure 5A** illustrates the presence of encephalotoxin in ventricular and lumbar CSF recovered from autopsy cases. **Figure 5B** illustrates the presence of encephalotoxin in ventricular and lumbar CSF recovered from living subjects. Data are expressed in terms of CSF volumes required to elicit death of cultured hippocampal neurons. As shown in the dose response curves (Figure 5A), small volumes of high toxin concentrations shift curves to the left, as found in those subjects with definite AD (diagnosis confirmed by autopsy). These data can also be expressed as ED₅₀s (volumes of CSF required to give 50% maximal killing). As shown in Figure 5B, a similar pattern was found in those subjects with probable AD (clinical diagnosis) who have small ED₅₀s (0.1 to 10 µl), followed by those in the MCI group with moderate values (10 to 200 µl). Importantly, various other diagnostic groups showed no detectable encephalotoxin (ED₅₀s > 1000 µl).

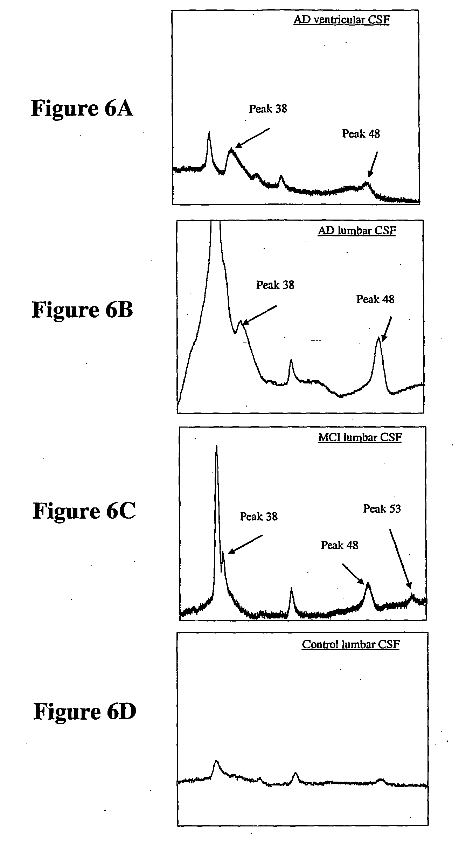

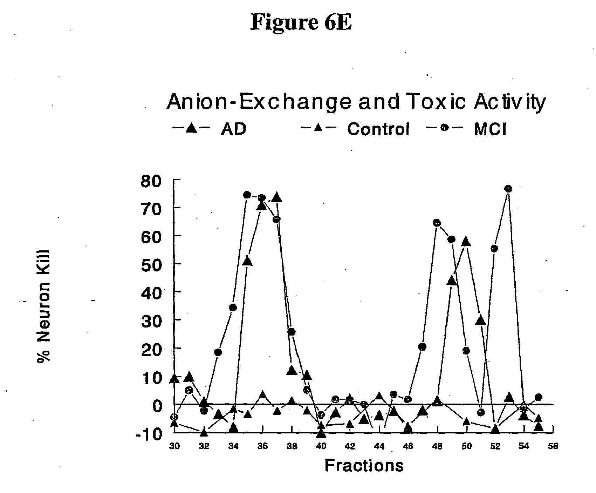

**Figure 6** shows PEAKs 38, 48, and 53 in CSF of AD (Panels A,B) and MCI (C), but not in normal elderly control (D) in anion exchange HPLC. These peaks were heparin lyase I sensitive (data not shown). As shown in Figure 6E, bioassays of these HPLC fractions confirm the same peaks are neurotoxic.

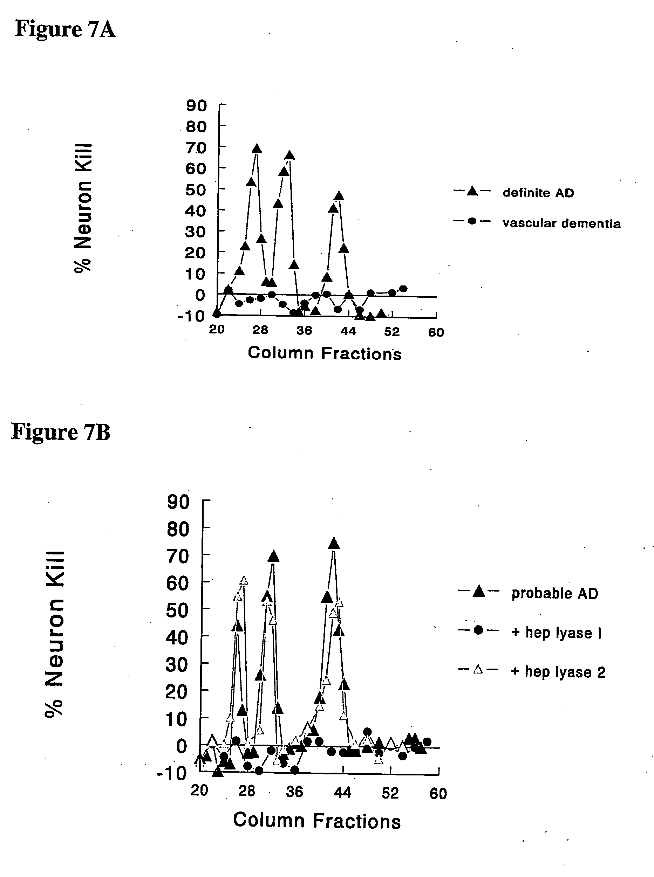

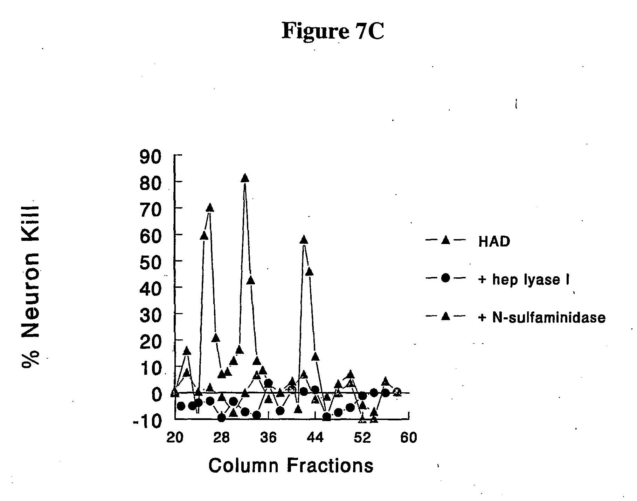

**Figure 7** shows that, in anion-exchange HPLC (linear gradient of 0 to 2.0 M NaCl over 90 min), 3 discrete peaks of neurotoxic activity are found in 100 µl of CSF from definite AD (Figure 7A), probable AD (Figure 7B), and HAD (Figure 7C). No toxic activity is recovered from vascular dementia (Figure 7A). Heparin lyase I and N-sulfaminidase, but not heparin lyase II, eliminate all toxin peaks.

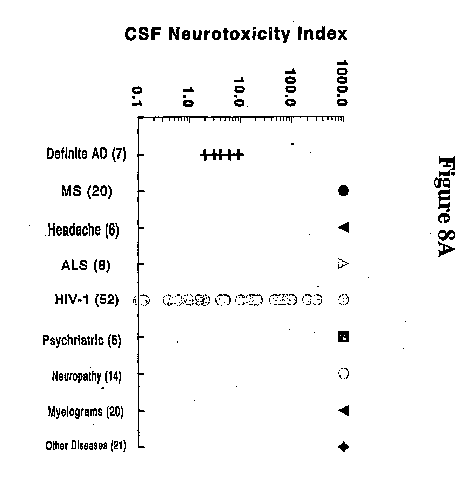

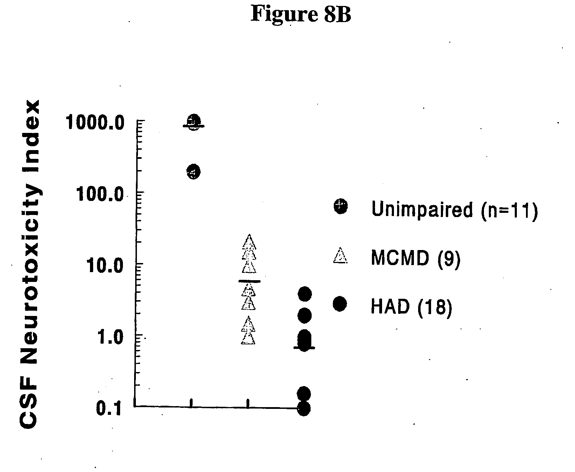

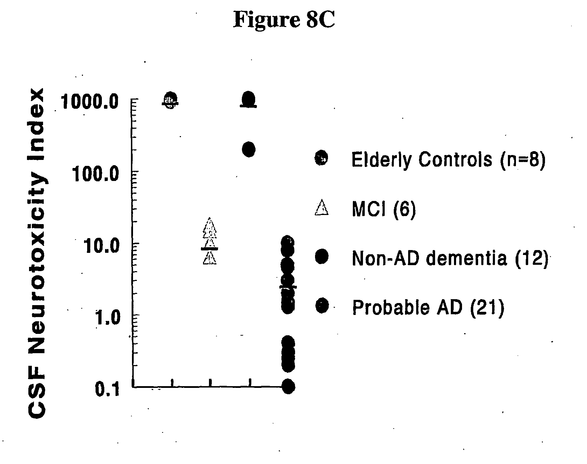

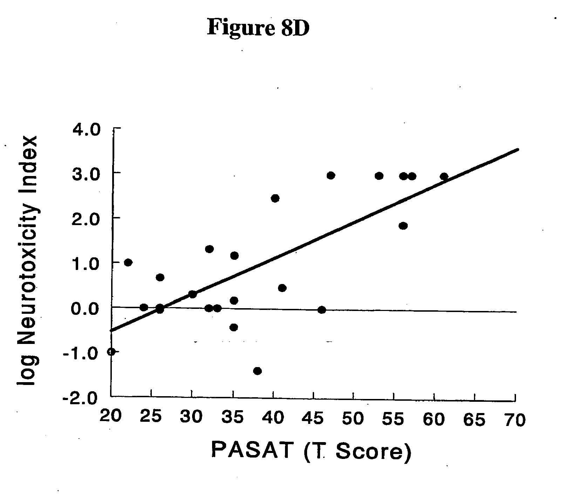

**Figure 8** illustrates the CSF Neurotoxicity Index, [calculated as value of equivalent volume of CSF to yield 50% of total killing effect upon a standardized rat hippocampal neuron culture assay] from cerebrospinal fluid (CSF) samples from a variety of neurological disorders. As shown in Figure 8A, samples from definite Alzheimer's disease (AD) and HIV-1 infection contain encephalotoxins. Cerebrospinal fluid obtained during routine lumbar myelogram (Myelograms) were from subjects without memory complaints. Neuropathy refers to subjects with cranial or peripheral nerve disorders while subjects with psychiatric diagnoses had no evidence of neurological disease. Other neurological diseases included fungal meningitis, neurosyphilis, multiple sclerosis (MS), and amyotrophic lateral sclerosis (ALS). Figure 8B compares CSF index scores with HIV-1(+) volunteers with no cognitive loss, mild cognitive motor dysfunction (MCMD), or HAD. Significant differences exist among MCMD and HAD, again supporting the pattern that more toxin is associated with greater degrees of cognitive impairment. Figure 8C compares CSF index scores for elderly volunteers with no cognitive loss, with MCI, with probable AD, or with non-AD dementia (caused by traumatic, vascular, or ethanol injury). MCI shows a consistent and significantly elevated level of encephalotoxin above other forms of dementia. Bars show median values. Figure 8D compares Neurotoxicity Index values vs. T scores for the paced auditory serial-addition test (PASAT, a sensitive measure of information processing.) As shown, a significant linear relationship exists between CSF Neurotoxicity Index and this cognitive measure (n=26; p< 0.0001; correlation coefficient = 0.74).

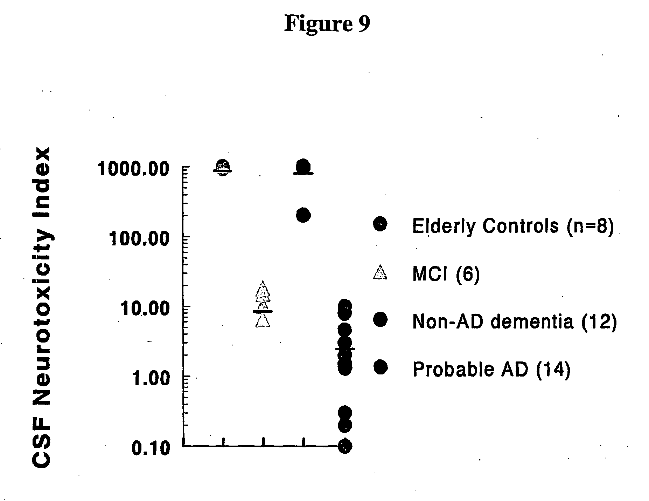

**Figure 9** shows a comparison of CSF Neurotoxicity Index scores of CSF from elderly subjects. Probable AD and MCI show significant toxin levels with an overlap in distribution of values. CSF neurotoxin levels clearly separate AD pathology from other categories common to the aged. (Bar = mean values.)

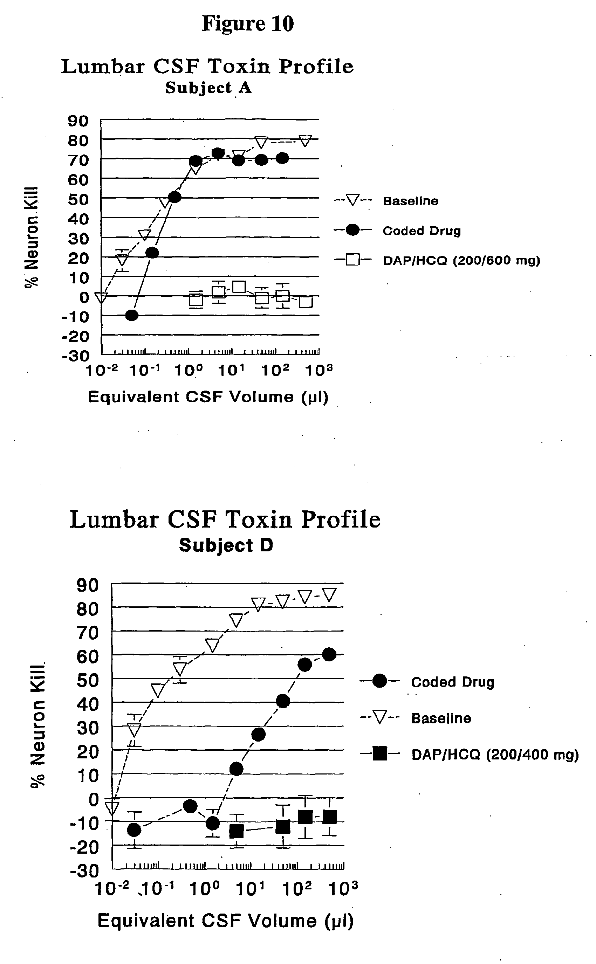

**Figure 10** shows two examples of drug effects upon CSF encephalotoxin levels. Single drug treatment (identity of drugs remains coded) failed to offer full suppression of toxin (i.e., shifted Index scores to a normal range of >100 as noted in Table 1) after a 6-week trial. In contrast, DAP/HCQ for 6 weeks provided complete inhibition of toxin production in all subjects tested to date (5 of 5).

As used herein, the term "about" refers to an approximation of a stated value within an acceptable range. Preferably the range is +/- 10% of the stated value.

Definite AD was diagnosed at autopsy using consensus neuropathological criteria (The NIA-Reagan Working Group. Consensus recommendations for the postmortem diagnosis of Alzheimer's disease. (1997) Neurobiol. Aging, 18:S1). The clinical definition for probable AD followed consensus recommendations (McKhann et al. (1984) Neurology 34:939) with impairment defined as psychometric performance falling at least 2 standard deviations (SD) below mean normative mean values in Learning/Memory [measured by the Wechsler Memory Scale-III Logical Memory Subtest, Hopkins Verbal Learning Test-Revised, or Brief Visual Memory Test-Revised, and 2 SD below normative mean on at least one test within the following cognitive domains: Attention/Information Processing [Verbal Sustained Attention Test, Symbol. Digit Modalities Test, Wechsler Adult Intelligence Test-III Digit Span, Trails A Test, and Paced Auditory Serial-Addition Test (PASAT)], Orientation (Orientation questions), Language [Naming and Category Fluency, FAS Test], Executive Function [Wisconsin Card Sort Test and Trials B Test]. Subjects with MCI are defined as those without dementia but who show amnestic features including a memory complaint confirmed by an informant and a memory impairment measured at least 1.5 SD below normative mean values using the same testing battery as for AD.

The clinical definitions for HIV-related cognitive impairments followed consensus recommendation (Working Group of American Academy of Neurology AIDS Task Force (1992) Neurology, 41:778) with subjects showing no evidence for other etiologies. Measured impairment for HIV-associated dementia (HAD) fell 2.5 SD below normative means in one domain or 2 SD in at least two domains on any of the following tests: Learning/Memory, Language, Attention/Information Processing, Abstraction/Problem Solving, and Motor Abilities [Grooved Pegboard]. Subjects with mild cognitive-motor dysfunction (MCMD) are defined as those falling 1.5 SD below mean normative values in any test in at least two cognitive domains or 2.0 SD below mean values in a single domain.

As used herein, "loss of cognition" or variants thereof refer to a decline in at least one of information processing, attention, learning, information retrieval, and overall loss of intellectual function. Loss of cognition may be measured by any method known in the art, including, for example, Attention/Information Processing [Verbal Sustained Attention Test, Symbol Digit Modalities Test, Wechsler Adult Intelligence Test-III Digit Span, Trails A Test, and Paced Auditory Serial-Addition Test (PASAT)], Orientation (Orientation questions), Language [Naming and Category Fluency, FAS Test], Executive Function [Wisconsin Card Sort Test and Trials B Test], Learning/Memory, Abstraction/Problem Solving, Motor Abilities [Grooved Pegboard], and Hopkins Verbal tests. A subject at risk for loss of cognition has no measurable loss of cognition but has a greater chance for loss of cognition than the average population. For example, a first-degree relative of an Alzheimer's disease patient is at risk for loss of cognition.

As used herein, the term "contact" or "contacting" means bringing together, either directly or indirectly, a compound into physical proximity to a molecule of interest. Contacting may occur, for example, in any number of buffers, salts, solutions, or in a cell or cell extract.

The term "peptide bond" means a covalent amide linkage formed by loss of a molecule of water between the carboxyl group of one amino acid and the amino group of a second amino acid.

The term "saccharide" or "saccharide unit" includes oxidized, reduced or substituted saccharides. Saccharides of this invention include, but are not limited to, ribose, arabinose, xylose, lyxose, allose, altrose, glucose, mannose, fructose, gulose, idose, galactose, talose, ribulose, sorbose, tagatose, gluconic acid, glucuronic acid, glucaric acididuronic acid rhamnose, fucose, N-acetyl glucosamine, N-acetyl galactosamine, N-acetyl neuraminic acid, sialic acid, N-sulfated glucosamine (GlcNS), 2-sulfated iduronic acid (IdoA2S), derivatives of saccharides such as acetals, amines; and phosphorylated sugars, oligosaccharides, as well as open chain forms of various sugars, and the like. "Oligosaccharide" refers to a molecule having two or more saccharide units.

The term "purified", when used to describe the state of the neurotoxin of the invention, refers to a neurotoxin substantially free of other cellular material. "Substantially free" refers to at least about 60% or about 70%, more preferably at least about 80% or about 90%, and most preferably at least about 95%, about 98%, or about 100% free of other cellular materials.

The prodromic phase of pathology of neurodegenerative or neuro-inflammatory disease is defined herein as that stage in the disease during which the clinical manifestations of cognitive, behavioral, or social impairment have not yet reached a diagnostic threshold for MCI (amnesic features with memory testing 1.5 SD below normative mean) or AD (2.SD below normative means in memory and at least one other cognitive domain). By this definition, the prodromic phase would encompass, for example, the clinically-defined Cognitive Impairment, No Dementia (CIND) population (Toukko et al. (2001) Int. Psychogeriatr., Supp. 1:183-202), the at-risk asymptomatic population described by Hom ((1994) J. Clin. Exp. Neurol., 16: 568-576), the Age-Associated Memory Impairment (AAMI, Goldman et al. (2001) Alz. Dis. Assoc. Dis., 15: 72-79), the subclinical cohort of the Farmington study (Elias et al. (2000) Arch. Neurol. 57: 808-813) or a preclinical AD population defined identified at autopsy (Price et al. (2001) Arch. Neurol., 58: 1395-1402). In general, all these groups show memory test values (verbal, episodic memory) at about 1 SD below normative mean scores adjusted for age, education, and ethnicity. Overall, populations at-risk for AD showed longitudinal declines at a rate of about 0.3 to 0.6 SD per year from normalized memory test scores tests.

The signaling cascade involved in the neurodegenerative diseases addressed by the present invention comprises events including (1) mononuclear phagocyte activation; (2) mononuclear phagocyte release of encephalotoxin; and (3) the toxic effect of encephalotoxins on neurons. Neurotoxicity of a mononuclear phagocyte induced by a mononuclear phagocyte activator may be inhibited or inactivated by an agent referred to herein as a neurotoxin inhibitor or inactivator.

A mononuclear phagocyte is an immune cell which has a single nucleus and the ability to engulf particles, also known as phagocytosis. Mononuclear phagocytes are found in blood and body tissues, including the central nervous system and brain, and include, for example, microglia cells, monocytes, macrophages, histiocytes, dendritic cells, precursor cells of microglia, precursor cells of monocytes, precursor cells of macrophages, microglia-like cell lines, macrophage-like cell lines, or cell lines modified to express microglia-like surface molecules that are active in accordance with the above definition of mononuclear phagocyte. A neuron as defined in accordance with the present invention includes a neuron and neuron-like cell, which is a cell modified to express a N-methyl-D-aspartate receptor which neuron exhibits neuronal activity under typical normal, non-diseased state, conditions.

Mononuclear phagocyte activation initiates a process that causes the release of neurotoxins. Mononuclear phagocyte activation is also referred to herein as immune activation, markers of which are any process that renders a mononuclear phagocyte more dynamic and characterized by activities such as and not limited to increased movement, phagocytosis, alterations in morphology, and the biosynthesis, expression, production, or secretion of molecules, such as protein, associated with membranes including complement, scavengers, Aβ, and blood cell antigens, histocompatibility antigens for example. Production of molecules includes enzymes involved in the biosynthesis of bioactive agents such as nitric oxide synthetase, superoxide dismutase, small molecules such as eicosanoids, cytokines, free radicals and nitric oxide. Release of factors includes proteases, apolipoproteins such as apolipoprotein E, and cytokines such as interleukin-1, tumor necrosis factor as well as other molecules such as encephalotoxin and hydrogen peroxide.

Mononuclear phagocyte neurotoxicity or neuron toxicity refers to a process that leads to the injury, destruction, or death of neurons, which is measured by loss of metabolic function, release of intracellular material, penetration of impermeant dyes, reduction of cell number measured by biochemical or histological methods. For example, changes in biochemical markers such as loss of neurofilaments or synaptophysin or release of lactate dehydrogenase, or other evidence of cell injury such as penetration of impermanent dyes, including fluorescent nuclear dyes and trypan blue. These and other strategies for identifying cell injury, destruction or death, or measuring neuron function, are known to one skilled in the art and are contemplated by the present invention.

Neurotoxin is defined herein as a substance that injures, damages, or kills a neuron while sparing other central nervous system cells such as glia, for example. A neurotoxin interacts with neurons in such a way as to disrupt neuron function and survival. The possible actions of a neurotoxin on neurons, also referred to herein as neuronal damage, include inhibition or disruption of normal cell metabolism, including metabolism of glucose, the production of ATP, and maintenance of ion gradients across cell membranes including Na⁺, Ca²⁺, and K⁺ ion channels, the synthesis of proteins and nucleic acids, and mitochondrial respiration, and cell death.

Encephalotoxin as used herein refers to a class of neurotoxins having low molecular mass (< 2000 daltons), heat stability, resistance to proteases, and loss of activity upon exposure to nitrous acid, N-sulfamidase, glucosamine-6-sulfatase, and heparin lyase I. Encephalotoxins comprise at least one GlcNS residue. An encephalotoxin preferably has a molecular weight between about 700 and 1,900 daltons. The encephalotoxin preferably has 4 to 8 saccharide residues.

Encephalotoxin inactivators or inhibitors are agents which inactivate neurotoxin or inhibit the effects of neurotoxins that are released from activated mononuclear phagocytes. For purposes of the present invention, inhibit, inhibition, inactivate, inactivation, and variations thereof are used synonymously with reduce, suppress, retard, slow, and suspend. Inactivation or inhibition also refers to complete inhibition of the neurotoxin cascade such that the cascade is arrested, stopped, or blocked. Encephalotoxin inactivation includes reduction of neurotoxic activity by about 10%, 20%, 50%, more preferably about 80%, 90%, or 95%, and most preferably about 98%, 99%, or 100%. By way of example, a compound is an encephalotoxin inactivator if it reduces the neurotoxic activity of the encephalotoxin or increases neuron survival such that neurons otherwise at risk of damage upon exposure to the encephalotoxin are not damaged in the presence of the encephalotoxin and the compound. Preferably, more than about 10%, 20%, or 50% of the neurons at risk are not damaged by the encephalotoxin in the presence of the encephalotoxin inactivator. Even more preferably, about 80%, 90%, or 95%, and most preferably, about 98%, 99%, or 100% of the neurons at risk are not damaged by the encephalotoxin in the presence of the encephalotoxin inactivator. Preferable encephalotoxin inactivators of the invention include heparin lyase I, N-sulfaminidase, glucosamine-6-sulfatase, and nitrous acid. Nitrous acid preferably has a pH of about 1.5. More preferably, exposure to nitrous acid occurs at room temperature.

An effective amount of a mononuclear phagocyte and an activator is the amount of each normally resulting in an event in the cascade, but for the addition of an encephalotoxin inactivator. An effective amount will be known to a skilled artisan in view of the present disclosure and will vary depending on the use of a mononuclear phagocyte, neuron, activator or components, and the mammalian origin of the cells.

*In vitro* neurotoxicity assays of the invention detect the presence of encephalotoxin and inactivation thereof and employ cultures of neurons or neuron-like cell lines which have been modified to express N-methyl-D-aspartate receptors. The presence of neurotoxic activity, or a measure of neuron function or measure of neuron survival, will be determined by reduction in cell number, changes in biochemical markers such as loss of cell metabolic function, release of intracellular material penetration of impermeant dyes, such as and not limited to fluorescent nuclear dyes and trypan blue, loss of neurofilament or synaptophysin, release of lactate dehydrogenase, or other evidence of cell injury. Other methods of measuring neuron function include detecting the inhibition of normal cell metabolism including the disruption of glucose metabolism, ATP production, ion gradient maintenance across cell membranes, and protein synthesis, nucleic acid synthesis, and mitochondrial respiration. Reductions in an inflammatory marker or injury to a neuron by a test biological sample may be compared to a control. These and other strategies for identifying cell neurotoxicity or measuring neuron function, which may be displayed as cell injury, are known to one skilled in the art and are contemplated by the present invention.

Using the assay systems of the invention, it is possible to diagnose subjects at early, for example, pre-symptomatic or prodromic, stages of neurological disease. It is further possible, using the methods of the invention, to identify subjects or populations at risk for loss of cognition by detecting the encephalotoxin in a biological sample of a subject. The methods of the invention also allow monitoring of progression of neurological disease by detecting increases in encephalotoxin levels of a subject over time. The patients or subjects to be diagnosed in accordance with the present invention include and are not limited to mammals such as humans, primates such as and not limited to monkey, chimpanzee, and ape, rodents, such as and not limited to rat and mouse, guinea pig, dog, cat, rabbit, and pig. Biological samples in accordance with the methods of the invention include central nervous system tissue, such as brain or spinal cord tissue, or cerebrospinal fluid (CSF). The neurological diseases to be identified or monitored according to the invention include neurodegenerative and neuro-inflammatory diseases such as, but not limited to, Alzheimer's disease, Creutzfeldt-Jakob disease, HIV-1 associated dementia (HAD), Mild Cognitive Impairment, prion disease, mild cognitive/ motor dysfunction, acute stroke, acute trauma, neuro-AIDS, and immune-mediated brain inflammation.

The methods of the present invention include a neurotoxin assay of a biological sample of a patient, which can be used to diagnose a neurological disease or disorder or risk for loss of cognition in the subject. The methods of the present invention also may be used as an early detection method to identify individuals who are at risk for developing neurological diseases or disorders in view of their age, family history, early symptoms or other risk factors. For example, a biological sample, such as blood, spinal cord tissue, cerebrospinal fluid, or brain tissue, may be taken from a patient and evaluated with the encephalotoxin inactivators of the present invention, as described herein, to identify the presence of encephalotoxins in the patient or to identify patients who may suffer from a neurological disease. The patient's sample may be compared to a control to determine whether elevated levels of neurotoxins are present.

Similarly, the methods of the present invention employ the neurotoxin inactivators of the invention to monitor a patient's treatment or the rate of progression of a disease by determining the amount of neurotoxins that are present in the patient's system before and throughout treatment. The methods may also be used to monitor neurotoxin levels to allow for the adjustment of drug doses.

For example, the present invention provides methods for assaying the presence and level of encephalotoxin in a patient by contacting a biological sample of the patient with an encephalotoxin inactivator, such as heparin lyase I, N-sulfaminidase, glucosamine-6-sulfatase, or nitrous acid. Thereafter, the amount of inhibition in the presence of the inactivator is compared to a measured control. There is an increase of encephalotoxin in the subject when there is an increase in the encephalotoxin level compared to the control.

The present invention offers strategies for early detection of neurodegenerative disease or risk for loss of cognition, thereby allowing early intervention in disease progression. The following examples are illustrative only and are not intended to limit the scope of the invention.

### EXAMPLES

### Purification of encephalotoxin

Encephalotoxins were isolated from cerebrospinal fluid by HPLC sieving chromatrophy (TSK-GEL G2500PWXL column; 7.8 x 300mm from Tosoh Bioscience; Montgomeryville, PA) eluted with 2 M NaCl; by anion exchange HPLC (tandem ProPac PA1 columns 4 x 250 mm from Dionex Corp.; Sunnyvale, CA) with a linear gradient of 2 M NaCl over 180 min; or by adsorption chromatography (Oasis Cartridges, Waters) using the manufacturer's protocol.

### Structural characterization and inactivation of encephalotoxin

Structural characterization and inactivation of encephalotoxin (isolated by organic extractions, gel filtration, and sequential C18 HPLC from Aβ-stimulated microglial cell line BV2) was performed by various nitrous acid cleavage protocols. Neurotoxic activity was eliminated by nitrous acid treatment at pH 1.5 but not by other acid treatments at pH 4.0 or with hydrazinolysis (**Figure 1**). The results indicated that the internal structure of encephalotoxin contained at least one GlcNS residue. Encephalotoxin chemical structure was further examined by treatments with highly selective enzymes that attack heparin or heparan sulfate (HS) polymers. Traditionally, heparin lyase I acts primarily on heparin-containing GlcNS(1→4)IdoA2S sequences and heparin lyase III on HS primarily at a GlcNAc(1→4)IdoA or GlcNAc(1→4)GlcA sequence. (Generally, these enzymes require oligomers of at least 4 residues.) Finally, encephalotoxin was treated with sulfatases that are highly selective for O-sulfation sites at positions 2, 3, or 6 (found in HS and heparins) as well as N-sulfamidase which cleaves the N-sulfation site (**Figure 1**). Heparin lyase I [GlcNS(1→4)IdoA2S], but not heparin lyase III, inactivated encephalotoxin as did sulfatases that removed groups from O-6S and GlcNS. Additionally, chemical methods to modify terminal amines (acetylation, PFPA modification, etc.) suggested the presence of terminal amines, such as unsubstituted GlcN residues. Accordingly, encephalotoxin contains heparin-like oligosaccharides of at least 4 residues with GlcNS, IdoA2S, GlcN residues plus O-linked sulfation at position 6.

Molecular mass of the neurotoxin was estimated using a TSK-GW2500PXL with a linear sieving range from 300 to 3000 daltons. Commercially available heparan oligomers were used as standards. CSF samples (100 ul) from probable AD showed a minor peak and major peak of neurotoxic activity having low molecular weight ranging in size from about 700 to 1,900 daltons. These estimated molecular masses suggest oligosaccharides from about 4 to 8 residues in length (**Figure 2**).

### Neurotoxin bioassay

Cultured neurons prepared from rat hippocampus were used in toxicity studies. These cultures consist of process-bearing neurons (10-20% of total cell population) atop a bed of astroglia (>70%) mixed with microglia (5-10%). In order to eliminate microglia, cultures were exposed to saporin coupled to acetylated LDL at 10 µg/ml for 18 hours. At the end of 72 hrs, the cultures were fixed in 3% paraformaldehyde at room temperature for 12 hours and immuno-stained by overnight incubation with a mixture of anti-neurofilament antibodies (SMI-311, 1:150; RT-97, 1:150; Sternberger Monoclonals, Inc.; ) plus anti-MAP-2 (1:200; Boehringer Mannheim, 184959;) at 4°C in the presence of 2% horse serum and 0.3% Triton X-100 to delineate both neuronal cell bodies and neurites. Immuno-labeled cells per field were scored at 200X magnification using fluorescence microscopy. Neuron killing was expressed as % mean survival expressed in terms of parallel untreated control cultures after scoring at least 8 randomly selected fields for each of 3 coverslips.

1 ml of CSF was fractionated by adsorption chromatography, dried under vacuum, and reconstituted in artificial CSF comprising electrolytes, such as NaCl, and glucose. Increasing amounts of fractionated toxin (range 0.1 to 500 ul equivalents of original sample volume) were added to triplicate cultures. Results were plotted as volume vs. % neuron kill (with kill calculated as % loss of immuno-stained hippocampal neurons against untreated control cultures). Inactivation, for example, by heparin lyase I, N-sulfaminidase, glucosamine-6-sulfatase, or nitrous acid treatment, was used to confirm the presence of encephalotoxin for each CSF sample tested. As shown in **Figure 3**, high levels of toxin (curves shifted to left) for AD, intermediate levels (curve shifted to right) for MCI, and toxin-free (flat line) profiles were noted for samples taken from disease controls. In order to compare different populations, a CSF Neurotoxicity Index was developed to assign scores that reflect level of neurotoxin. This index was calculated as an ED₅₀ (the equivalent CSF volume that yields 50% of the maximal level of neuron killing). Using this measurement, high neurotoxin levels have low Index scores; for example, high toxin concentrations have low Index scores of about 1, intermediate levels at about 5 to 100, and normal elderly show values of 1000.

### Encephalotoxin chemical assay

Anion-exchange HPLC conditions for the detection of encephalotoxin were established (0.0-0.7 m NaCl gradient; ProPAK PA-1 column; 232 nm UV monitoring). The microglial cell line BV2 was exposed to human Aβ1-42 for 48 hr and the conditioned media fractioned by adsorption chromatography. Three biologically-active peaks (PEAKs 38, 48, and 53) were recovered that corresponded to 3 peaks detected by 232 nm (**Figure 4A**). All 3 peaks were sensitive to nitrous acid pH 1.5 and to heparin lyase I (data not shown). Importantly, none of these peaks were recovered from control cultures of unstimulated BV2 cells (**Figure 4B**).

### Encephalotoxin as CSF Biomarker for Neurodegenerative Disease

Using ventricular CSF from rapid autopsy cases, encephalotoxin was determined to be present in high concentrations in all CSFs from AD cases (confirmed by pathology), but not in cases from age-matched normals or ALS (**Figure 5**). Importantly, lumbar CSF taken from subjects with a clinical diagnosis of probable AD also showed a striking pattern, with very high Encephalotoxin concentrations measured as ED₅₀s of between 0.1 to 5 µl.

A research protocol was established to evaluate samples not only from elderly subjects with cognitive impairment, but also from other groups seen by our clinic neurologists. The latter populations consisted of various diagnostic categories, with the largest groups suffering from headache variants, multiple sclerosis, or non-AD dementia (vascular, trauma). [Neurotoxin assays on these latter populations were performed with subject consent on remnant aliquots of CSF acquired for other clinical indications.] Data obtained thus far from subjects show that all patients with probable AD have high levels of neurotoxin, with ED₅₀s for equivalent CSF volumes ranging from 0.5 to 15 µl (note that lower ED₅₀ volumes indicate higher toxin concentrations); elderly subjects with MCI had ED₅₀ of between 50 and 200 µl. The non-parametric Kruskal-Wallis one-way ANOVA for ranks showed neurotoxin levels significantly differed (as measured by ED₅₀s) among tested disease groups (probable AD, MCI, non-AD dementia, headache, and MS; p=0.000001). The Kruskal-Wallis multiple-comparison test showed that both AD and MCI neurotoxin levels were significantly greater than these levels found in MS, headache, or non-AD dementia (p<0.02 for all comparisons).

Overall, these observations revealed several important trends. First, subjects with probable AD had the highest toxin concentrations, falling within a narrow range, similar to that of ventricular CSF from AD autopsy cases. Second, severe cognitive impairment or dementia secondary to non-inflammatory mechanisms (vascular, post-trauma) did not show detectable amounts of encephalotoxin in the CSF. [While neurotoxin can be found in tissues damaged acutely after stroke or trauma, these neurotoxin levels dissipate as the acute inflammatory response dissipates (about 3 to 7 days post injury; Giulian et al. (1990) Ann. Neuro., 27: 33-42; Giulian et al. (1993) Stroke, 24: 84-93; Giulian (1993) Glia, 7: 102-110)]. And third, there appeared to be a trend of MCI subjects showing significant amounts of encephalotoxin, but only 1/10 to 1/100 as much total toxic activity as found in AD CSFs (**Figure 5**).

To determine whether oligosaccharides associated with encephalotoxin were also found in human CSF, encephalotoxin was isolated from CSF by adsorption chromatography and treated with the same heparin lyases, nitrous acid treatments, and sulfatases as used for microglia culture media. Ventricular CSF from AD cases and lumbar CSF from probable AD subjects demonstrated the same inactivation profiles (**Figure 1**), indicating that encephalotoxin in human disease contained heparin-like oligomers. Confirmation of the presence of such neurotoxic oligosaccharides came from anion-exchange HPLC, showing the presence of a neurotoxic PEAK 38 recovered from microglial encephalotoxin fractions. There were similarities between the CSF samples from AD and MCI by anion-exchange profiles (PEAKS 38 and 48) with an additional PEAK 53 in the MCI group (**Figure 6**) as noted in microglial cultures (**Figure 4**).

HPLC-profiles for ventricular cerebrospinal fluid of cases of definite AD were nearly identical to lumbar fluid samples from volunteers with probable AD (Figure 7B) and from those with HAD (**Figure 7C**). Enzymatic treatments by heparin lyase I and by N-sulfamindase eliminated all these peaks of neurotoxicity. Neuron-killing activity recovered by anion-exchange HPLC was insensitive to heparin lyase II (**Figure 7B**), proteases, or heparin lyase III treatments (data not shown).

In order to survey the prevalence of neurotoxin production in neurological disorders, the cerebrospinal fluid of subjects from various disease populations was examined. Neurotoxin concentrations, expressed as CSF Neurotoxicity Index scores [expressed as equivalent volume of CSF which yields 50% of a total neuron killing effect in a standardized rat hippocampal culture assay], show that only those subjects with definite AD (postmortem diagnosis; n=7) or HIV-1 infection (n=52) had detectable levels of CSF neurotoxin (**Figure 8A**). Neurologic disorders that can elicit chronic reactive immune responses, such as multiple sclerosis (MS; n=20), amyotrophic lateral sclerosis (ALS; n=8), or neuropathies (n=14), had no CSF neurotoxin. Similarly, subjects with psychiatric illness (n=5), with headache (n=6), or a variety of other neurological diseases (n=21; including fungal meningitis and neurosyphilis) are free of detectable neurotoxin. And finally, CSF samples obtained from volunteers undergoing routine myelography (n=20) contained no neurotoxin activity.
Neurotoxicity Index values for CSF in cases of definite AD ranged between 1 and 10 whereas a broader distribution appeared for the HIV(+) population (0.1 to 1000). To investigate the wider distribution of neurotoxin levels for the HIV-1(+) cohort, 7 coded lumbar CSF samples from the HIV-1(+) volunteers who had undergone extensive medical, neurological, and neuropsychological evaluations were obtained through the Texas unit of the National Neuro-AIDS Tissue Consortium (Morello et al. (2001) Neuropath. Appl. Neurobiol., 27:326-335). Neurotoxins are detected in those subjects with cognitive dysfunction (n=4) but not in those found to have normal cognition (n=3; Fisher's Exact Test, p=0.028). Low CSF Neurotoxicity Index scores were detected in HIV(+) subjects with HAD (range from 0.1 to 4.0); high Index scores were detected in HIV(+) subjects with little or no cognitive impairment (all > 200), and intermediate Index scores (1.0 to 21.0) were associated with HIV(+) subjects identified with mild cognitive-motor disorder (MCMD;Working Group of American Academy of Neurobiology AIDS Task Force (1992) Neurology, 41:778; Figure 8B). Significant differences between MCMD (median 7.3; mean +/- SE, 9.0 +/- 2.7; n=8) and HAD (0.1 median; 0.8 +/- 0.3; n=14) for Index values show a high confidence level (p=0.0001; Kruskal Wallis). The separation between HIV-1(+) subjects with MCMD group and those without cognitive impairment (median 1000.0; mean 900 +/- 99.7 µl; n=8) is also significant (p=0.001). The degree of HIV injury to the CNS reflects levels of CSF neurotoxin, implying causal relationships among cognitive impairment, stage of brain pathology, and the production of neuron poisons.

In order to determine whether neurotoxin levels also reflect cognitive decline in the aged population, CSF was obtained from elderly volunteers with Mild Cognitive Impairment of the amnestic type (MCI; objective memory deficit, but without dementia; Bischkopf et al. (2002) Acta Psychiatr. Scand., 106:403-414; n=6), a condition of impaired memory thought to reflect an early stage of AD (DeKosky et al. (2003) Science, 302:830). Comparison of subjects with MCI to elderly volunteers serving as controls (>70 years old and free of memory complaints; n=8) showed marked differences between the groups (Figure 8C). The Neurotoxicity Index scores for MCI ranges from about 7 to 20 (median 10.0; mean 11.5 +/- 1.6; n=6) and are significantly lower than those measured for elderly controls (all > 1000; n=8; Kruskal-Wallis; p=0.0005). Index scores for volunteers with probable AD (defined by clinical criteria) show a range of values from 0.1 to 10 (median 1.7, mean 3.0 +/- 0.8; n=21). Probable AD and MCI values are also significantly different (p=0.0111; Kruskal-Wallis), further evidencing an association between levels of CSF encephalotoxin and stage of brain pathology underlying cognitive dysfunction. Importantly, other forms of dementia lacking chronic brain inflammation, such as those secondary to trauma, alcoholism, or vascular injury, produce little or no detectable CSF neurotoxin (median 1000; mean 933.0 +/- 66.6; n=12). These observations are in agreement with CSF encephalotoxin values found in autopsy-confirmed cases for definite AD (Figure 8A) and for vascular dementia (Figure 8A).

In order to classify groups according to CSF neurotoxin concentrations, discriminant analyses were applied to three diagnostic categories for HIV(+) subjects and three categories for the elderly. As shown in Table 1, the CSF Neurotoxicity Index accurately predicts which HIV(+) volunteers will have little or no impairment in cognition (cut-off >100) from among those groups with MCMD (1-100) or HAD (<1). Similarly, the Index correctly separates subjects with non-Alzheimer's dementia (cut-off >100) from the elderly with MCI or AD. A cut-off value of >100 also predicts with 100% accuracy those elderly without memory complaints (see Figure 8C).

**Table 1. Cut-off Values for CSF Neurotoxicity Index According to Disease Category**

| **A.** | | | |
|---|---|---|---|
| | **Neurotoxicity Cut-off Values** | | |
| **Diagnostic Group** | **>100** | **1-100** | **<1** |
| **HIV (+) unimpaired (n=11)** | **100%** | **0%** | **0%** |
| **Mild Cognitive Motor Dysfunction (MCMD) (n=9)** | **0%** | **100%** | **0%** |
| **HAD (n=14)** | **0%** | **21%** | **79%** |

| **B.** | | | |
|---|---|---|---|
| | **Neurotoxicity Cut-off Values** | | |
| **Diagnostic Group** | **>100** | **>4-100** | **<4** |
| **Non-AD dementia (n=20)** | **100%** | **0%** | **0%** |
| **MCI (n=6)** | **0%** | **100%** | **0%** |
| **AD (n=20)** | **0%** | **33%** | **67%** |

### CSF Encephalotoxin as a Biomarker for Progression of Disease Pathology

Data from 164 subjects showed that all patients with AD have high levels of neurotoxin in the CSF with ED₅₀s for equivalent CSF volumes ranging from 0.5 to 15 µl. Elderly subjects with mild cognitive impairment had levels between 50 and 200 µl. Subjects with various other neurological disorders, including neurodegenerative diseases, had no detectable toxicity (ED₅₀s > 1000 µl); vascular and post-trauma non-AD dementia also had no toxic activity. HIV-1 (+) subjects demonstrated a wide range of toxin concentrations (ED₅₀s ranging from 0.6 µl to >1000 µl).

CSF from 40 HIV-1 (+) individuals was examined. The level of toxicity was associated with the degree of cognitive impairment. For example, HIV-1 (+) subjects with normal cognition showed ED₅₀s>1000 µl, while those with moderate to severe cognitive defects produced neurotoxin levels of 0.6 to 5 µl, similar to the range found for AD subjects with established dementia.HIV-1 (+) subjects with mild to moderate cognitive impairments had intermediary levels of CSF neurotoxin with ED₅₀s ranging from 10 to 300 µl.

The Neurotoxicity Index in a variety of diagnostic groups was measured and compared against definite AD (n=7; defined by neuropathologic diagnosis using ventricular CSF obtained post mortem). As shown in Figure 8A, there is a striking difference between AD and other diagnostic categories lacking measurable toxin (Index scores of 1000), thus evidencing the value of the Neurotoxicity Index across a broad population. Furthermore, as shown in Figure 9, CSF encephalotoxin levels are clearly different among elderly without memory complaints or non-AD dementia (vascular, post traumatic, neurosyphillis) when compared to MCI (with amnestic features) or probable AD populations (using NINCDS-ADRDA diagnostic criteria). Discriminant analyses (Table 1B) established cut-off Neurotoxicity Index values for AD at <4 and for MCI at 4 to 100, providing the ability to correctly assign diagnosis based upon toxin values for MCI or probable AD against other groups. The underlying pathological process advances as a subject moves from a pre-symptomatic state to mild impairment (MCI with a 1.5 SD drop below norms of a standardized memory test) and then to a more advanced stage with dementia (AD with a 2 SD drop below norms in memory and at least one other domain). Earlier stages of disease prior to significant memory loss (stages before diagnosis of MCI) involve the neuron-damaging immune cascade which is detectable by the presence of CSF encephalotoxin. This subclinical stage is the prodromic phase of AD pathology.

Correlation between toxin levels and clinical manifestations of disease progression has been elucidated. MCI and mild AD subjects (MMSE > 20; CDR < 1) having CSF encephalotoxin were subjected to a detailed neuro-cognitive battery. Simple linear regression analyses were carried out comparing Neurotoxicity Index values with T scores from sets of standardized tests representing major cognitive domains. (T scores are normalized to 50 with 10 as SD; raw scores are adjusted for age, gender, ethnicity, and education level). As shown in Table 2, a highly significant correlation exists between Index scores and abnormal memory; that is, higher concentrations of toxin are found in those subjects with greater memory deficits while other cognitive domains (abstraction, language, processing speed) are not.

**Table 2.**

| **Cognitive Domain** | **p=** | **corr coef =** |
|---|---|---|
| | | |
| **Executive Function** | | |
| **Wisconsin Card Sort** | NS | |
| **Trails B** | NS | |
| | | |

| **Memory/Learning** | | |
|---|---|---|
| **Hopkins Verbal** | 0.007 | 0.784 |
| **WMS-III** | 0.001 | 0.800 |

| **Information Processing** | | |
|---|---|---|
| **digit symbol** | NS | |
| **symbol** search | NS | |
| Trails A | NS | |
| | | |

| **Language** | | |
|---|---|---|
| **FAS** | NS | |

Table 3 compares CSF Neurotoxin Index values and T scores for specific cognitive tests among HIV-1(+) volunteers (n=33). Confidence levels are based upon linear regression analyses and show that cognitive defects with domains of attention/information processing and learning/memory are closely associated with the amount of CSF encephalotoxin, while language and motor function are not. Prior to analysis, the Neurotoxicity Index was log transformed so that data would follow an approximate normal distribution.

**Table 3**

| **Cognitive Domain** | **Test** | **Confidence Level (p=)** | **Correlation Coefficient** |
|---|---|---|---|
| **Abstraction/Problem Solving** | | | |
| Visual Reasoning | Wisconsin Card Sort | 0.010 | 0.462 |
| Visual-Motor Sequencing | Trails Making B | 0.003 | 0.514 |

| **Language** | | | |
|---|---|---|---|
| Verbal Fluency | FAS | NS | |

| **Learning and Memory** | | | |
|---|---|---|---|
| Auditory Word List | Hopkins Verbal Learning Test | 0.001 | 0.529 |
| Visual Simple Figures | Brief Visual Memory Test | 0.001 | 0.531 |
| Word Recall | Hopkins-Delayed Recall | 0.009 | 0.444 |
| Figure Recall | Brief Visual - Delayed Recall | 0.002 | 0.523 |

| **Attention/Information Processing** | | | |
|---|---|---|---|
| Auditory Series | PASAT | 0.000 | 0.735 |
| Number-Symbol Translation | WAIS III Digit Symbol | 0.014 | 0.427 |
| Visual Patterns | WAIS III Symbol Search | 0.000 | 0.574 |
| Visual-Motor Scanning | Trails Making A | 0.011 | 0.442 |

| **Motor Abilities** | | | |
|---|---|---|---|
| Psychomotor Sp eed/Dexterity | Grooved Pegboard | NS | |

### Examine effects of suppressive agents for microglia upon CSF encephalotoxin levels

Use of encephalotoxin as a biomarker for monitoring drug treatment and disease progression was examined in a 6-week double-blind randomized study comparing several drugs against placebo with the primary endpoint as change in encephalotoxin levels in the CSF. Despite the masking of group assignments, a striking pattern was identified, as shown by representative data in **Figure 10**. Although some subjects receiving coded drugs showed reduction in toxin levels by about 10-fold, such decreases did not shift subjects into the range of Index scores found among normal elderly (that is, Index scores remained below the MCI cut-off values of 100). These data suggested that none of the active drugs used in this trial were adequately dosed to provide complete neuroprotection. The persistence of significantly abnormal encephalotoxin concentrations made it unlikely that a single drug trial would alter the clinical course of AD.

A secondary endpoint was used to assess the ability of drug treatments to reduce Aβ-induced toxicity in cultured blood monocytes. It was found in animal studies that blood mononuclear phagocytes reflect brain microglial responses to Aβ. Accordingly, drug responses in cultures of blood monocytes having a baseline toxicity measure in enrolled subjects prior to drug treatments were examined after entry into the masked single drug trial. Study of 76 monocyte samples with measurement of Aβ-induced toxicity have shown the following:
1) in some cases a single drug (identity masked) completely suppress Aβ-activation of blood monocytes;
2) single drugs that suppress blood monocytes offer only a partial inhibition of CSF encephalotoxin levels;
3) *ex vivo* studies using blood monocytes from subjects without evidence of drug suppression demonstrated exquisite sensitivity to DAP/HCQ combinations at 1/10 doses.

Various modifications of the invention in addition to those shown and described herein will be apparent to one skilled in the art from the foregoing description.

## Claims

1. A method of diagnosing a neurological disease or risk for loss of cognition in a subject comprising detecting an encephalotoxin in a biological sample comprising cerebrospinal fluid, spinal cord tissue or brain tissue of said subject, wherein said detecting comprises contacting said biological sample of said subject with neurons both in the presence of and in the absence of an encephalotoxin inactivator and comparing neuron survival in the presence of said encephalotoxin inactivator relative to neuron survival in the absence of said encephalotoxin inactivator, a decrease in neuron survival in the absence of said encephalotoxin inactivator being indicative of said neurological disease, the encephalotoxin being an oligosaccharide comprising at least one glucosamine having N-sulfation and 06-sulfation; said encephalotoxin lacking peptide bonds; and said encephalotoxin having a molecular weight of less than 2000 daltons; the encephalotoxin inactivator being heparin lyase I, N-sulfaminidase, glucosamine-6-sulfatase, or nitrous acid solution.

2. The method of claim 1, wherein said step of comparing neuron survival comprises comparison of the ED50 of said encephalotoxin in the presence of said encephalotoxin inactivator relative to the ED50 of the encephalotoxin in the absence of said encephalotoxin inactivator, wherein a lower ED50 of the encephalotoxin in the absence of said encephalotoxin inactivator relative to the ED50 of the encephalotoxin in the presence of said encephalotoxin inactivator is indicative of said neurological disease.

3. A method of diagnosing a neurological disease or risk for loss of cognition in a subject comprising detecting an encephalotoxin in a biological sample comprising cerebrospinal fluid, spinal cord tissue or brain tissue of said subject, wherein said step of detecting comprises comparing light absorbance of said biological sample in the presence of an encephalotoxin inactivator to light absorbance of said biological sample in the absence of said encephalotoxin inactivator, said light absorbance being measured following HPLC adsorption chromatography, and an increased absorbance in the absence of said encephalotoxin inactivator being indicative of said neurological disease, the encephalotoxin being an oligosaccharide comprising at least one glucosamine having N-sulfation and 06-sulfation, lacking peptide bonds, and having a molecular weight of less than 2000 daltons.

4. The method of claim 3 wherein the encephalotoxin inactivator is heparin lyase I, N-sulfaminidase, glucosamine-6-sulfatase or nitrous acid solution.

5. The method of claim 1 or claim 4, wherein said nitrous acid solution has a pH of 1.5.

6. A method of monitoring treatment of a neurological disease in a subject comprising comparing encephalotoxin level in a first and second biological sample of said subject, said first and second biological samples each comprising cerebrospinal fluid, spinal cord tissue or brain tissue, wherein said first biological sample is taken from said subject at an earlier timepoint than said second biological sample, wherein said second biological sample is taken from said subject following treatment of said neurological disorder, and wherein said encephalotoxin level is measured by light absorbance of said biological sample, said light absorbance being measured following HPLC adsorption chromatography, and an increased light absorbance of said second biological sample being indicative of progression of said neurological disease, the encephalotoxin being an oligosaccharide comprising at least one glucosamine having N-sulfation and 06-sulfation; said encephalotoxin lacking peptide bonds; and said encephalotoxin having a molecular weight of less than 2000 daltons.

7. The method of claim 6, wherein said first biological sample is taken from said subject following treatment of said neurological disease.

8. A method of monitoring progression of neurological disease in a subject comprising detecting an increase in encephalotoxin level in said subject over time, wherein said step of detecting comprises measuring an increased light absorbance of an encephalotoxin in a first biological sample of said subject relative to light absorbance of an encephalotoxin of a second biological sample of said subject, wherein said first and second biological samples each comprise cerebrospinal fluid, spinal cord tissue or brain tissue, and second biological sample is taken from said subject before said first biological sample, wherein said light absorbance is measured following HPLC adsorption chromatography, wherein said encephalotoxin comprises an oligosaccharide comprising at least one glucosamine having N-sulfation and 06-sulfation, lacks peptide bonds, and has a molecular weight of less than 2000 daltons, said increased light absorbance being indicative of progression of said neurological disease.

9. The method of any of claims 3, 6 or 8 wherein said light absorbance is measured at a wavelength of 232 nanometers.

10. A method for monitoring progression of neurological disease in a subject comprising detecting an increase in encephalotoxin level in said subject over time, wherein said step of detecting comprises contacting a first biological sample of said subject with neurons, contacting a second biological sample of said subject with neurons, wherein said first and second biological samples each comprise cerebrospinal fluid, spinal cord tissue or brain tissue, and detecting decreased neuron survival in the presence of said second biological sample, wherein said second biological sample is taken at a later timepoint than said first biological sample; wherein said encephalotoxin comprises an oligosaccharide comprising at least one glucosamine having N-sulfation and 06-sulfation, lacks peptide bonds, and has a molecular weight of less than 2000 daltons; and wherein said decreased neuron survival is indicative of progression of said neurological disease.

11. The method of claim 10 wherein one of said biological samples is taken during the prodromic phase of said neurological disease.

12. A method of monitoring treatment of a neurological disease in a subject comprising detecting an increase in encephalotoxin level in said subject over time, wherein said step of detecting comprises contacting a first biological sample of said subject with neurons, contacting a second biological sample of said subject with neurons, wherein said first and second biological samples each comprise cerebrospinal fluid, spinal cord tissue or brain tissue, and detecting decreased neuron survival in the presence of said second biological sample, wherein said second biological sample is taken at a later timepoint than said first biological sample and following treatment of said neurological disease; wherein said encephalotoxin comprises an oligosaccharide comprising at least one glucosamine having N-sulfation and 06-sulfation, lacks peptide bonds, and has a molecular weight of less than 2000 daltons; and wherein said decreased neuron survival is indicative of progression of said neurological disease.

13. The method of claim 10 or 12, wherein said decreased neuron survival is detected by comparing the ED50 of said first biological sample with the ED50 of the second biological sample, wherein a lower ED50 of the second biological sample relative to the ED50 of the first biological sample is indicative of progression of said neurological disease.

14. The method of any preceding claim, wherein said neurological disease is HIV-1-associated dementia (HAD), neuro-AIDS, Creutzfeldt-Jakob Disease, Mild Cognitive Impairment, prion disease, mild cognitive/ motor dysfunction, acute stroke, acute trauma, or Alzheimer's disease (AD).

15. The method of any preceding claim, wherein said encephalotoxin has a molecular weight of 700 to 1900 daltons.

16. The method of any preceding claim, wherein said encephalotoxin comprises 4 to 8 saccharide units.

17. The method of any preceding claim, wherein said subject is human, primate, bovine, equine, canine, feline, porcine, or rodent.

18. The method of claim 17 wherein said subject is human.

## Patentansprüche

1. Verfahren zur Diagnostizierung einer neurologischen Erkrankung oder des Risikos von Gedächtnisverlust bei einem Subjekt, enthaltend die Ermittlung eines Encephalotoxins in einer biologischen Probe, die Cerebrospinalflüssigkeit, spinales Rückenmarksgewebe oder Gehirngewebe besagten Subjekts enthält, wobei besagte Ermittlung das Kontaktieren besagter biologischer Probe besagten Subjekts mit Neuronen beinhaltet sowohl in Anwesenheit als auch in Abwesenheit eines Encephalotoxininaktivators und Vergleichen des Überlebens der Neuronen in Anwesenheit besagten Encephalotoxinaktivators relativ zum Überleben der Neuronen in Abwesenheit besagten Encephalotoxininaktivators, wobei die Abnahme des Überlebens der Neuronen in Abwesenheit besagten Encephalotoxinaktivators als Anzeige besagter neurologischer Erkrankung dient, und wobei das Encephalotoxin ein Oligosaccherid ist. welches wenigstens ein Glucosamin mit N-Sulfation und O6-Sulfation enthält; besagtes Encephalotoxin enthält keine Peptidbonds und besagtes Encephalotoxin besitzt ein Molekulargewicht von weniger als 2000 Daltons; der Encephalotoxinaktivator ist Heparinlyase I, N-Sulaminidase, Glucosamin-6-Suliatase oder Salpetersäurelösung.

2. Verfahren nach Anspruch 1, wobei der Schritt des Vergleichs der überlebenden Neuronen den Vergleich von ED50 besagten Encephalotoxins in Anwesenheit besagten Encephalotoxininaktivators relativ zu ED50 des Encephalotoxins in Abwesenheit besagten Encephalotoxininaktivators beinhaltet, wobei ein niedrigeres ED50 des Encephalotoxins in Abwesenheit besagten Encephalotoxinaktivators relativ zu dem ED50 des Encephalotoxins in Anwesenheit des besagten Encephalotoxininaktivators indikativ für besagte neurologische Erkrankung ist.

3. Verfahren zur Diagnostizierung einer neurologischen Erkrankung oder des Risikos auf Gedächtnisverlust eines Subjekts, welches die Ermittlung eines Encephalotoxins in einer biologischen Probe, welche Cerebrospinalflüssigkeit, spinales Rückenmarksgewebe oder Hirngewebe besagten Subjekts enthält, wobei besagter Schritt der Ermittlung den Vergleich der Lichtabsorbtion besagter biologischer Probe in Anwesenheit eines Encephalotoxininaktivators zur Lichtabsorbtion besagter biolgischer Probe in Abwesenheit besagten Encephalotoxininaktivators enthält, wobei besagte Lichtabsorbtion gemessen wird als Folge der HPLC Adsorptionschromatographie und eine vermehrte Absorbtion in Abwesenheit besagten Encephalotoxininaktivators Anzeige für besagte neurologische Erkrankung ist, wobei das Encephalotoxin ein Oligosaccherid ist, welches wenigstens ein Glucosamin mit N-Sulfation und O6-Sulfation enthält, sowie Peptidbonds nicht enthält und eine Molekulargewicht von weniger als 2000 Daltons besitzt.

4. Verfahren nach Anspruch 3, wobei der Encephalotoxininaktivator Heparinlyase 1 ist, N-Sulfaminidase, Glucosamin-6-Sulfatase oder Salpetersäure.

5. Verfahren nach Anspruch 1 oder Anspruch 4, wobei besagte Salpetersäure einen PH-Wert von 1.5 hat.

6. Verfahren zur Überwachung der Behandlung einer neurologischen Erkrankung in einem Subjekt enthaltend den Vergleich des Encephalotoxinniveaus in einer ersten und zweiten biologischen Probe besagten Subjekts, besagte erste und zweite biologische Proben jeweils enthaltend Cerebrospinalflüssigkeit, spinales Rückenmarksgewebe oder Gehirngewebe, wobei besagte erste biologische Probe von besagtem Subjekt zu einem früheren Zeitpunkt als besagte zweite biologische Probe genommen wird und wobei besagte zweite biologische Probe von besagtem Subjekt nach der Behandlung besagter neurologischer Erkrankung genommen wird und wobei besagtes Encephalotoxinniveau gemessen wird durch Lichtabsorbtion besagter biologischer Probe, besagte Lichtabsorbtion wird gemessen nach HPLC Adsorbtionschromatographie und eine vermehrte Lichtabsorbtion besagter zweiter biologischer Probe als Anzeige des Fortschritts besagter neurologischer Erkrankung dient, wobei das Encephalotoxin ein Oligosaccharid ist, welches wenigstens ein Glucosamin enthält mit N-Sulfation und O6-Sulfation; besagtes Encephalotoxin enthält keine Peptidbonds und besagtes Encephalotoxin hat ein Molekulargewicht von weniger als 2000 Daltons.

7. Verfahren nach Anspruch 6, wonach besagte erste biologische Probe aus besagtem Subjekt nach der Behandlung besagter neurologischer Erkrankung genommen wird.

8. Verfahren zur Überwachung des Fortschritts neurologischer Erkrankung eines Subjekts enthaltend das Ermitteln einer Zunahme des Encephalotoxinniveaus besagten Subjekts über die Zeit, wobei besagter Schritt zur Ermittlung die Messung einer vermehrten Lichtabsorbtion eines Encephalotoxins in einer ersten biologischen Probe besagten Subjekts relativ zur Lichtabsorbtion eines Encephalotoxins einer zweiten biologischen Probe besagten Subjekts enthält, wobei besagte erste und zweite biologische Proben jeweils Cerebrospinalfluid, spinales Rückenmarksgewebe oder Gehirngewebe enthält und die zweite biologische Probe aus besagtem Subjekt vor der ersten biologischen Probe entnommen wird, wobei besagte Lichtabsorbtion gemessen wird nach der HPLC Adsorptionschromatographie, wobei besagtes Encephalotoxin ein Oligosaccherid enthält welches wenigstens ein Glucosamin mit N-Sulfation und O6-Sulfation enthält, keine Peptidbonds und ein Molekulargewicht von weniger als 2000 Daltons, wobei besagte Lichtabsorbtion die Anzeige des Fortschritts besagter neurologischer Erkrankung ist.

9. Verfahren nach einem der Ansprüche 3, 6 oder 8 wobei besagte Lichtabsorbtion gemessen wird bei einer Wellenlänge von 232 Nanometern.

10. Verfahren zur Überwachung des Fortschritts neurologischer Erkrankung eines Subjekts enthaltend das Ermitteln einer Zunahme des Encephalotoxinniveaus in besagtem Subjekt über der Zeit, wobei besagter Schritt zur Ermitteln das Inkontaktbringen einer ersten biologischen Probe besagten Subjekts mit Neuronen und das Inkontaktbringen einer zweiten biologischen Probe besagten Subjekts mit Neuronen enthält, wobei besagte erste und zweite biologische Proben jeweils Cerebrospinalfluid, spinales Rückenmarksgewebe oder Gehirngewebe enthalten, und die Ermittlung verringertes Überleben der Neuronen in Anwesenheit besagter zweiter biologischer Probe umfasst, wobei besagte zweite biologische Probe zu einem späteren Zeitpunkt als besagte erste biologische Probe genommen wird; wobei besagtes Encephalotoxin ein Oligosaccherid enthält, welches wenigstens ein Glucosamin aufweist mit N-Sulfation und O6-Sulfation sowie keine Peptidbindung und ein Molekulargewicht von weniger als 2000 Daltons; und wobei besagte Abnahme überlebender Neuronen als Anzeige des Fortschritts besagter neurologischer Erkrankung dient.

11. Verfahren nach Anspruch 10, wobei eine besagter biologischer Proben während der Prodromalphase besagter neurologischer Erkrankung genommen wird.

12. Verfahren zur Überwachung der Behandlung einer neurologischen Erkrankung eines Subjekts enthaltend die Ermittlung einer Zunahme des Encephalotoxinniveaus in besagtem Subjekt über die Zeit, wobei besagter Schritt der Ermittlung das Inkontaktbringen einer ersten biologischen Probe besagten Subjekts mit Neuronen enthält, das Inkontaktbringen einer zweiten biologischen Probe besagten Subjekts mit Neuronen, wobei besagte erste und zweite biologische Proben jeweils ein Cerebrospinalfluid, spinales Rückenmarksgewebe oder Hirngewebe enthalten und die Abnahme des Überlebens von Neuronen in Anwesenheit besagter zweiter biologischer Probe, wobei besagte zweite biologische Probe zu einem späteren Zeitpunkt als besagte erste biologische Probe genommen wird und nach der Behandlung besagter neurologischer Erkrankung; wobei besagtes Enzephalotoxin ein Oligosaccherid enthält, welches wenigstes ein Glucosamin aufweist mit N-Sulfation und 06-Sulfation, keine Peptidbonds und ein Molekulargewicht von weniger als 2000 Daltons besitzt; und wobei besagte Abnahme überlebender Neuronen als Anzeige des Fortschritts besagter neurologischer Erkrankung dient.

13. Verfahren nach Anspruch 10 oder 12, wobei besagte Abnahme überlebender Neuronen ermittelt wird durch Vergleich des ED50 besagter erster biologischen Probe mit dem ED50 besagter zweiter biologischer Probe und wobei ein niedrigeres ED50 besagter zweiter biologischer Probe relativ zu dem ED50 besagter erster biologischer Probe als Anzeige des Fortschritts besagter neurologischer Erkrankung dient.

14. Verfahren nach einem der vorangegangenen Ansprüche, wobei besagte neurologische Erkrankung HIV-1-begleitete Demenz (HAD) ist, Neuro-AIDS, Creutzfeldt-Jakob Erkrankung, geringe Gedächtnisverschlechterung, Prionerkrankung, geringe Gedächtnis/motorische Dysfunktion, akuter Schlaganfall, akutes Trauma oder Alzheimererkrankung (AD).

15. Verfahren nach einem der vorangegangenen Ansprüche, wobei besagtes Encephalotoxin ein Molekulargewicht von 700 bis 1900 Daltons aufweist.

16. Verfahren nach einem der vorangegangenen Ansprüche, wobei besagtes Encephalotoxin 4 - 8 Saccharideinheiten enthält.

17. Verfahren nach einem der vorangegangenen Ansprüche, wobei besagtes Subjekt von humaner, primatenartiger, boviner, pferdeartiger, hundeartiger, katzenartiger, schweineartiger oder nagetierartiger Art ist.

18. Verfahren nach Anspruch 17, wobei besagtes Subjekt humaner Art ist.

## Revendications

1. Procédé de diagnostic d'une maladie neurologique ou d'un risque de perte cognitive chez un sujet comprenant la détection d'une encéphalotoxine dans un échantillon biologique comprenant du liquide céphalorachidien, du tissu de la moelle épinière ou du tissu cérébral dudit sujet, où ladite détection comprend la mise en contact dudit échantillon biologique dudit sujet avec des neurones en présence et en l'absence d'un inactivateur de l'encéphalotoxine et la comparaison de la survie neuronale en présence dudit inactivateur de l'encéphalotoxine avec la survie neuronale en l'absence dudit inactivateur de l'encéphalotoxine, une diminution de la survie neuronale en l'absence dudit inactivateur de l'encéphalotoxine indiquant ladite maladie neurologique, l'encéphalotoxine étant un oligosaccharide comprenant au moins une glucosamine ayant une N-sulfatation et une 06-sulfatation ; ladite encéphalotoxine étant dépourvue de liaisons peptidiques ; et ladite encéphalotoxine ayant une masse moléculaire inférieure à 2000 daltons ; l'inactivateur de l'encéphalotoxine étant l'héparine lyase I, la N-sulfaminidase, la glucosamine-6-sulfatase ou une solution d'acide nitreux.

2. Procédé selon la revendication 1, où ladite étape de comparaison de la survie neuronale comprend la comparaison de la DE50 de ladite encéphalotoxine en présence dudit inactivateur de l'encéphalotoxine avec la DE50 de l'encéphalotoxine en l'absence dudit inactivateur de l'encéphalotoxine, où une DE50 plus faible de l'encéphalotoxine en l'absence dudit inactivateur de l'encéphalotoxine par rapport à la DE50 de l'encéphalotoxine en présence dudit inactivateur de l'encéphalotoxine indique ladite maladie neurologique.

3. Procédé de diagnostic d'une maladie neurologique ou d'un risque de perte cognitive chez un sujet comprenant la détection d'une encéphalotoxine dans un échantillon biologique comprenant du liquide céphalorachidien, du tissu de la moelle épinière ou du tissu cérébral dudit sujet, où ladite étape de détection comprend la comparaison de l'absorbance de la lumière dudit échantillon biologique en présence d'un inactivateur de l'encéphalotoxine avec l'absorbance de la lumière dudit échantillon biologique en l'absence dudit inactivateur de l'encéphalotoxine, ladite absorbance de la lumière étant mesurée après chromatographie d'adsorption HPLC et une élévation de l'absorbance en l'absence dudit inactivateur de l'encéphalotoxine indiquant ladite maladie neuronale, l'encéphalotoxine étant un oligosaccharide comprenant au moins une glucosamine ayant une N-sulfatation et une 06-sulfatation, étant dépourvue de liaisons peptidiques et ayant une masse moléculaire inférieure à 2000 daltons.

4. Procédé selon la revendication 3 où l'inactivateur de l'encéphalotoxine est l'héparine lyase I, la N-sulfaminidase, la glucosamine-6-sulfatase ou une solution d'acide nitreux.

5. Procédé selon la revendication 1 ou la revendication 4, où ladite solution d'acide nitreux a un pH de 1,5.

6. Procédé de surveillance du traitement d'une maladie neurologique chez un sujet comprenant la comparaison du taux d'encéphalotoxine dans un premier et un second échantillon biologique dudit sujet, lesdits premier et second échantillons biologiques comprenant chacun du liquide céphalorachidien, du tissu de moelle épinière ou du tissu cérébral, où ledit premier échantillon biologique est prélevé chez ledit sujet antérieurement au dit second échantillon biologique, où ledit second échantillon biologique est prélevé chez ledit sujet après le traitement dudit trouble neurologique, et où ledit taux d'encéphalotoxine est mesuré par l'absorbance de la lumière dudit échantillon biologique, ladite absorbance de la lumière étant mesurée après chromatographie d'adsorption HPLC et une élévation de l'absorbance de la lumière dudit second échantillon biologique indiquant la progression de ladite maladie neurologique, l'encéphalotoxine étant un oligosaccharide comprenant au moins une glucosamine ayant une N-sulfatation et une 06-sulfatation ; ladite encéphalotoxine étant dépourvue de liaisons peptidiques ; et ladite encéphalotoxine ayant une masse moléculaire inférieure à 2000 daltons.

7. Procédé selon la revendication 6, où ledit premier échantillon biologique est prélevé chez ledit sujet après traitement de ladite maladie neurologique.

8. Procédé de surveillance de la progression d'une maladie neurologique chez un sujet comprenant la détection d'une élévation du taux d'encéphalotoxine chez ledit sujet au fil du temps, où ladite étape de détection comprend la mesure de l'élévation de l'absorbance de la lumière d'une encéphalotoxine dans un premier échantillon biologique dudit sujet par rapport à l'absorbance de la lumière d'une encéphalotoxine d'un second échantillon biologique dudit sujet, où lesdits premier et second échantillons biologiques comprennent chacun du liquide céphalorachidien, du tissu de moelle épinière ou du tissu cérébral et ledit second échantillon biologique est prélevé chez ledit sujet avant ledit premier échantillon biologique, où ladite absorbance de la lumière est mesurée après chromatographie d'adsorption HPLC, où ladite encéphalotoxine comprend un oligosaccharide comprenant au moins une glucosamine ayant une N-sulfatation et une 06-sulfatation, est dépourvue de liaisons peptidiques et a une masse moléculaire inférieure à 2000 daltons, ladite élévation de l'absorbance de la lumière indiquant une progression de ladite maladie neurologique.

9. Procédé selon l'une quelconque des revendications 3, 6 ou 8, où ladite absorbance de la lumière est mesurée à une longueur d'onde de 232 nanomètres.

10. Procédé de surveillance de la progression d'une maladie neurologique chez un sujet comprenant la détection d'une élévation du taux d'encéphalotoxine chez ledit sujet au fil du temps, où ladite étape de détection comprend la mise en contact d'un premier échantillon biologique dudit sujet avec des neurones, la mise en contact d'un second échantillon biologique dudit sujet avec des neurones, où lesdits premier et second échantillons biologiques comprennent chacun du liquide céphalorachidien, du tissu de moelle épinière ou du tissu cérébral, et la détection d'une diminution de la survie neuronale en présence dudit second échantillon biologique, où ledit second échantillon biologique est prélevé ultérieurement au dit premier échantillon biologique ; où ladite encéphalotoxine comprend un oligosaccharide comprenant au moins une glucosamine ayant une N-sulfatation et une 06-sulfatation, est dépourvue de liaisons peptidiques et a une masse moléculaire inférieure à 2000 daltons ; et où ladite diminution de la survie neuronale indique une progression de ladite maladie neurologique.

11. Procédé selon la revendication 10, où l'un desdits échantillons biologiques est prélevé durant la phase prodromique de ladite maladie neurologique.

12. Procédé de surveillance du traitement d'une maladie neurologique chez un sujet comprenant la détection d'une élévation du taux d'encéphalotoxine chez ledit sujet au fil du temps, où ladite étape de détection comprend la mise en contact d'un premier échantillon biologique dudit sujet avec des neurones, la mise en contact d'un second échantillon biologique dudit sujet avec des neurones, où lesdits premier et second échantillons biologiques comprennent chacun du liquide céphalorachidien, du tissu de moelle épinière ou du tissu cérébral, et la détection d'une diminution de la survie neuronale en présence dudit second échantillon biologique, où ledit second échantillon biologique est prélevé ultérieurement au dit premier échantillon biologique et après traitement de ladite maladie neurologique ; où ladite encéphalotoxine comprend un oligosaccharide comprenant au moins une glucosamine ayant une N-sulfatation et une 06-sulfatation, est dépourvue de liaisons peptidiques et a une masse moléculaire inférieure à 2000 daltons ; et où ladite diminution de la survie neuronale indique une progression de ladite maladie neurologique.

13. Procédé selon la revendication 10 ou 12, où ladite diminution de la survie neuronale est détectée par comparaison de la DE50 dudit premier échantillon biologique avec la DE50 dudit second échantillon biologique, où une DE50 plus faible dudit second échantillon biologique par rapport à la DE50 dudit premier échantillon biologique indique une progression de ladite maladie neuronale.

14. Procédé selon l'une quelconque des revendications précédentes, où ladite maladie neurologique est la démence associée à VIH-1 (HAD), le neuro-sida, la maladie de Creutzfeld-Jakob, le déficit cognitif léger, les maladies transmises par les prions, le dysfonctionnement cognitif/moteur léger, l'ictus aigu, le traumatisme aigu ou la maladie d'Alzheimer (AD).

15. Procédé selon l'une quelconque des revendications précédentes, où ladite encéphalotoxine a une masse moléculaire de 700 à 1900 daltons.

16. Procédé selon l'une quelconque des revendications précédentes, où ladite encéphalotoxine comprend 4 à 8 unités saccharidiques.

17. Procédé selon l'une quelconque des revendications précédentes, où ledit sujet est un humain, un primate, un bovin, un équin, un canin, un félin, un porcin ou un rongeur.

18. Procédé selon la revendication 17, où ledit sujet est un humain.
